# EUROPÄISCHE PATENTSCHRIFT

(11) **EP 1 636 169 B1**
(45) Veröffentlichungstag und Bekanntmachung des Hinweises auf die Patenterteilung: **02.06.2010**
(21) Anmeldenummer: 04740107.0
(22) Anmeldetag: 21.06.2004
(51) Int. Cl.: C07C 213/08, C07C 217/62

(54) **VERFAHREN ZUR DEHYDRATISIERUNG SUBSTITUIERTER 4-DIMETHYLAMINO-2-ARYL-BUTAN-2-OL-VERBINDUNGEN UND VERFAHREN ZUR HERSTELLUNG SUBSTITUIERTER DIMETHYL-(3-ARYL-BUTYL)-AMIN-VERBINDUNGEN MITTELS HETEROGENER KATALYSE**
METHOD FOR DEHYDRATING SUBSTITUTED 4-DIMETHYLAMINO-2-ARYL-BUTAN-2-OL COMPOUNDS AND METHOD FOR PRODUCING SUBSTITUTED DIMETHYL-(3-ARYL-BUTYL)-AMINE COMPOUNDS BY MEANS OF HETEROGENEOUS CATALYSIS
PROCEDE DE DESHYDRATATION DE COMPOSES 4-DIMETHYLAMINO-2-ARYL-BUTANE-2-OL SUBSTITUES, ET PROCEDE DE FABRICATION DE COMPOSES DIMETHYL-(3-ARYL-BUTYL)-AMINE SUBSTITUES, PAR CATALYSE HETEROGENE

(30) Priorität: 23.06.2003 DE 10328316
(43) Veröffentlichungstag der Anmeldung: 22.03.2006
(73) Patentinhaber: Grünenthal GmbH, 52078 Aachen (DE)
(72) Erfinder: JAGUSCH, Utz-Peter, 52066 Aachen (DE); HÖLDERICH, Wolfgang, 67227 Frankenthal (DE); WISSLER, Monika, 01109 Dresden (DE)
(86) Internationale Anmeldenummer: PCT/EP2004/006666
(87) Internationale Veröffentlichungsnummer: WO 2005/000788

(56) Entgegenhaltungen:
- EP-A- 0 799 819
- DE-A- 2 254 929
- R. BUSSAS ET.AL.: "Ene Reaction Mechanisms. 1. Chirality Transfer to the Enophile 4-Methyl-N-sulfinylbenzenesulfonamide" J.ORG.CHEM., Bd. 48, 1983, Seiten 2828-2832, XP002305386

## Beschreibung

Die vorliegende Erfindung betrifft ein Verfahren zur Dehydratisierung substituierter 4-Dimethylamino-2-aryl-butan-2-ol-Verbindungen sowie Verfahren zur Herstellung substituierter Dimethyl-(3-aryl-butyl)-amin-Verbindungen mittels heterogener Katalyse.

Opioide, wie beispielsweise Morphin, werden seit vielen Jahren in der Schmerztherapie eingesetzt, obwohl sie eine Reihe von Nebenwirkungen, wie z.B. Sucht, Abhängigkeit, Atemdepression, gastro-intestinale Hemmwirkung und Obstipation, hervorrufen. Sie können daher nur unter besonderen Vorsichtsmaßnahmen über einen längeren Zeitraum und in höheren Dosierungen eingenommen werden (Goodman, Gilman, The Pharmacological Basis of Therapeutics, Pergamon Press, New York 1990).

Im Einklang mit dem hohen Bedarf an einer für den Patienten zufriedenstellenden Schmerztherapie steht die Suche nach neuen, gut wirksamen und verträglichen Schmerzmitteln im Mittelpunkt der medizinischen Forschung.

Mit der Entwicklung substituierter Dimethyl-(3-aryl-but-3-enyl)-amin-verbindungen, wie sie z.B. in der EP 0 799 819 oder J. org. Chem. 48, 1983, Seiten 2828 - 2832, beschrieben werden, ist es gelungen, neue Schmerzmittel zur Verfügung zu stellen, die sich durch eine sehr gute Wirksamkeit auszeichnen und die keine oder gegenüber den herkömmlichen Schmerzmitteln zumindest deutlich verminderte Nebenwirkungen zeigen.
Die Herstellung dieser Verbindungen erfolgt durch Dehydratisierung von entsprechend substituierten 4-Dimethylamino-2-aryl-butan-2-ol-Verbindungen, die eine tertiäre Alkoholfunktion aufweisen, mit Säure, insbesondere Ameisensäure oder Salzsäure.
Nachteilig bei diesem Verfahren ist, daß die zur Dehydratisierung eingesetzte Säure anschließend durch Neutralisation und ggf. mehrmalige Extraktion aus der Reaktionsmischung abgetrennt werden muß.

Das anfallende Salz kann zu Korrosionsprozessen an den Anlagenteilen führen und wirkt sich - wie auch die anfallenden Abwässer- negativ auf Umweltbilanz und Produktionskosten des Verfahrens aus.

Eine weitere Wirkstoffklasse mit exzellenter analgetischer Wirksamkeit und sehr guter Verträglichkeit stellen die substituierten Dimethyl-(3-aryl-butyl)-aminverbindungen dar, die u.a. aus der EP 0 693 475 bekannt sind.
Die Herstellung dieser pharmazeutischen Wirkstoffe erfolgt ebenfalls ausgehend von tertiären Alkoholen, in dem diese zunächst in die entsprechende Chloridverbindung übergeführt und anschließend mit Zinkborhydrid, Zinkcyanoborhydrid oder Zinncyanoborhydrid reduziert werden. Dieses Verfahren hat den Nachteil, daß die Herstellung der Chloridverbindung unter Verwendung vergleichsweise aggressiver Chlorierungsmittel wie Thionylchlorid erfolgt. Des weiteren verläuft dieses Verfahren nicht in allen Fällen mit zufriedenstellender Ausbeute.

Aufgabe der vorliegenden Erfindung war es daher, ein Verfahren zur Eliminierung der tertiären Alkoholfunktion aus substituierten 4-Dimethylamino-2-aryl-butan-2-ol-Verbindungen zur Verfügung zu stellen, mit dem die entsprechend substituierten Dimethyl-(3-aryl-but-3-enyl)-amin-verbindungen unter umweltschonenden Bedingungen in guten Ausbeuten erhalten werden und wodurch auch die Herstellung entsprechend substituierter Dimethyl-(3-aryl-butyl)-aminverbindungen mit vereinfachter Verfahrensführung in guten Ausbeuten gelingt.

Erfindungsgemäß wird diese Aufgabe durch die Bereitstellung der nachstehend beschriebenen Verfahren zur Dehydratisierung substituierter 4-Dimethylamino-2-arylbutan-2-ol-Verbindungen der nachstehend angegebenen allgemeinen Formel I sowie zur Herstellung substituierter Dimethyl-(3-aryl-butyl)-amin-Verbindungen der nachstehend angegebenen allgemeinen Formel III, ggf. unter Isolierung von substituierten Dimethyl-(3-aryl-but-3-enyl)-Verbindungen der nachstehend angegebenen allgemeinen Formel II gelöst. Die Verbindungen der allgemeinen Formeln II und III werden vorzugsweise als pharmazeutische Wirkstoffe in Arzneimitteln eingesetzt und eignen sich insbesondere zur Schmerzbekämpfung.

Ein Gegenstand der vorliegenden Erfindung ist daher ein Verfahren zur Dehydratisierung wenigstens einer substituierten 4-Dimethylamino-2-aryl-butan-2-ol-Verbindung der allgemeinen Formel I, 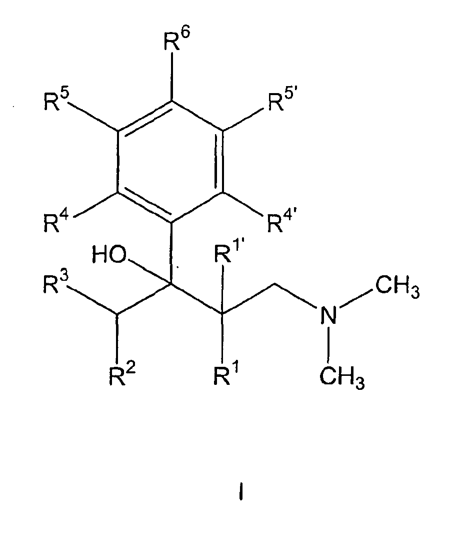 worin
R¹ H oder C₁₋₅-Alkyl bedeutet,
R^{1'} H oder C₁₋₅-Alkyl bedeutet,
R² H oder C₁₋₅-Alkyl bedeutet,
R³ H oder C₁₋₅-Alkyl bedeutet,
R⁴, R^{4'}, R⁵, R^{5'}, R⁶, gleich oder verschieden, jeweils für H, OH, C₁₋₄-Alkyl, OC₁₋₄-Alkyl teil- oder perfluoriertes C₁₋₄-Alkyl, teil- oder perfluoriertes O-C₁₋₄-Alkyl, O-(CH₂)ₙ-Phenyl mit n = 1, 2 oder 3, F, Cl oder OR⁸ stehen,
oder zwei benachbarte Reste R⁴ und R⁵, R⁵ und R⁶, R⁶ und R^{5'} oder R^{5'} und R^{4'} für eine Gruppe -OCH=CHO-, -CH=C(R⁹)-O-, -CH=C(R⁹)-S- oder -CH=CH-C(OR¹⁰)=CH- als Teil eines Ringes stehen, mit der Maßgabe, daß die jeweils anderen Reste R⁶, R^{5'}
und R^{4'}, R⁴, R^{5'} und R^{6'}, R⁴, R⁵ und R^{4'} bzw. R⁴, R⁵ und R⁶ die vorstehend genannte Bedeutung haben,
R⁸ CO-C₁₋₅-Alkyl, PO(O-C₁₋₄-Alkyl)₂, CO-C₆H₄-R¹¹, CO(O-C₁₋₅-Alkyl), CO-CHR¹²-NHR¹³, CO-NH-C₆H₃-(R¹⁴)₂ oder eine unsubstituierte oder substituierte Pyridyl-, Thienyl-, Thiazoyl- oder Phenylgruppe bedeutet,
R⁹ H oder C₁₋₄-Alkyl bedeutet,
R¹⁰ H oder C₁₋₃-Alkyl bedeutet,
R¹¹ OC(O)-C₁₋₃-Alkyl in ortho-Stellung oder -CH₂-N-(R₁₅)₂ in meta- oder para-Stellung, wobei R¹⁵ jeweils C₁₋₄-Alkyl oder beide Reste R¹⁵ zusammen mit dem verbrückenden Stickstoffatom einen 4-Morpholino-Rest bilden,
R¹² und R¹³, gleich oder verschieden, jeweils für H, C₁₋₆-Alkyl oder C₃₋₈-Cycloalkyl stehen,
oder R¹² und R¹³ zusammen -(CH₂)₃₋₈ als Teil eines Rings bedeuten,
R¹⁴ H, OH, C₁₋₇Alkyl, teil- oder perfluoriertes C₁₋₇-Alkyl, OC₁₋₇-Alkyl, Phenyl, O-Aryl, F oder Cl mit der Maßgabe, daß die Reste R¹⁴ gleich oder verschieden sind,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates,
gemäß dem wenigstens eine Verbindung der allgemeinen Formel I unter heterogener Katalyse zu einer substituierten Dimethyl-(3-aryl-but-3-enyl)-amin-Verbindung der allgemeinen Formel II, 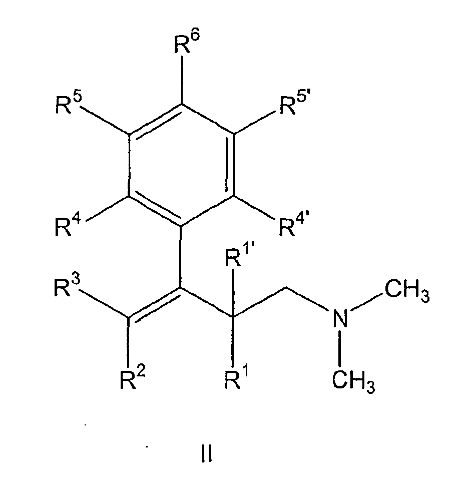 worin R¹, R¹', R², R³, R⁴, R^{4'}, R⁵, R^{5'} und R⁶ jeweils die vorstehend genannte Bedeutung haben, ggf. jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, als Endprodukt dehydratisiert, und dieses ggf. isoliert und ggf. gereinigt wird.

Die nach dem vorstehend beschriebenen Verfahren erhaltenen substituierten Dimethyl-(3-aryl-but-3-enyl)-amin-verbindungen der allgemeinen Formel II können nach üblichen, dem Fachmann bekannten Verfahren zu substituierten Dimethyl-(3-aryl-butyl)-amin-verbindungen der nachstehenden allgemeinen Formel III umgesetzt werden.

Ein weiterer Gegenstand der vorliegenden Erfindung ist daher ein Verfahren zur Herstellung wenigstens einer substituierten Dimethyl-(3-aryl-butyl)-amin-verbindung der allgemeinen Formel III, 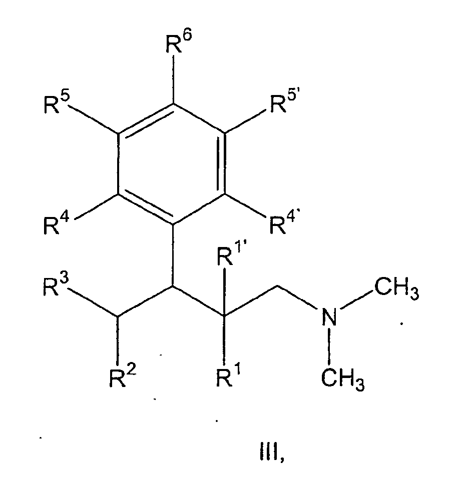 worin die Reste R¹, R¹', R², R³, R⁴, R^{4'}, R⁵, R^{5'} und R⁶ jeweils die vorstehend genannte Bedeutung haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, gemäß dem wenigstens eine substituierte 4-Dimethylamino-2-aryl-butan-2-ol-verbindung der allgemeinen Formel I 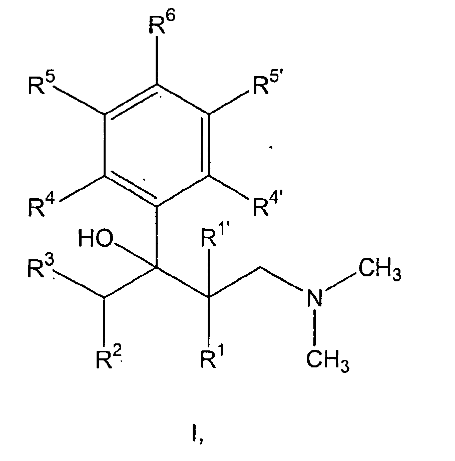 worin die Reste R¹, R¹', R², R³, R⁴, R^{4'}, R⁵, R^{5'} und R⁶ jeweils die vorstehend genannte Bedeutung haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, unter heterogener Katalyse zu einer substituierten Dimethyl-(3-aryl-but-3-enyl)-amin-Verbindung der allgemeinen Formel II, 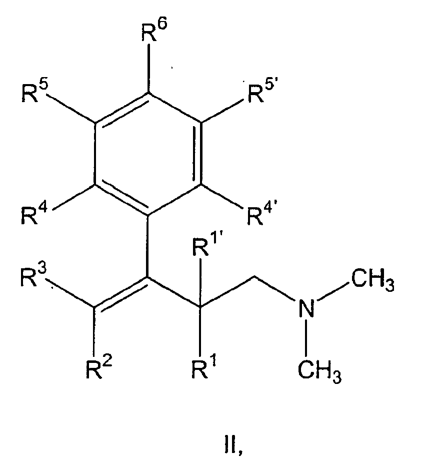 worin die Reste R¹ R¹', R², R³, R⁴, R^{4'}, R⁵, R^{5'} und R⁶ jeweils die vorstehend genannte Bedeutung haben, ggf. jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, als Zwischenprodukt dehydratisiert wird, dieses Zwischenprodukt ggf. isoliert, ggf. gereinigt und zu einer substituierten Dimethyl-(3-aryl-butyl)-amin-Verbindung der allgemeinen Formel III als Endprodukt umgesetzt wird.

Bevorzugt kommen in den erfindungsgemäßen Verfahren substituierte 4-Dimethylamino-2-aryl-butan-2-ol-Verbindungen der allgemeinen Formel Ia, 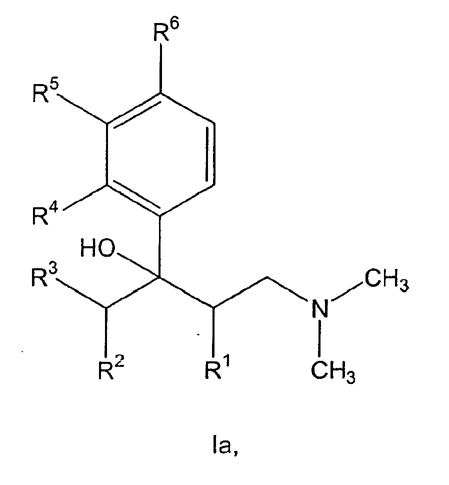 zum Einsatz, in denen
R¹ C₁₋₅-Alkyl ist,
R² H oder C₁₋₅-Alkyl bedeutet
R³ H oder C₁₋₅-Alkyl bedeutet,
R⁴ H, OH, C₁₋₄-Alkyl, O-C₁₋₄-Alkyl, O-Benzyl, CF₃, O-CF₃, Cl, F oder OR⁸ ist,
R⁵ H, OH, C₁₋₄-Alkyl, O-C₁₋₄-Alkyl, O-Benzyl, CHF₂, CF₃, O-CF₃, Cl, F oder OR⁸ ist,
R⁶ H, OH, C₁₋₄-Alkyl, O-C₁₋₄-Alkyl, O-Benzyl, CF₃, O-CF₃, Cl, F oder OR⁸ bedeutet,
mit der Maßgabe, dass zwei der Reste R⁴, R⁵ oder R⁶ H sind,
oder R⁴ und R⁵ zusammen eine Gruppe -CH=C(R⁹)-O- oder -CH=C(R⁹)-S- als Teil eines Ringes bedeuten mit der Maßgabe, dass R⁶ H ist, oder R⁵ und R⁶ zusammen
eine Gruppe -CH=CH-C(OR¹⁰)=CH- als Teil eines Ringes bedeuten mit der Maßgabe, dass R⁴ H ist,
R⁸ CO-C₁₋₅-Alkyl, PO(O-C₁₋₄-Alkyl)₂, CO-C₆H₄-R₁₁, CO(O-C₁₋₅-Alkyl),
CO-CHR¹²-NHR¹³, CO-NH-C₆H₃-(R¹⁴)₂ oder eine unsubstituierte oder substituierte Pyridyl-, Thienyl-, Thiazoyl- oder Phenylgruppe bedeutet,
R⁹ H oder C₁₋₄-Alkyl bedeutet,
R¹⁰ H oder C₁₋₃-Alkyl bedeutet,
R¹¹ OC(O)-C₁₋₃-Alkyl in ortho-Stellung oder -CH₂-N-(R¹⁵)₂ in meta- oder para-Stellung, wobei R¹⁵ C₁₋₄-Alkyl ist oder beide Reste R¹⁵ zusammen mit dem verbrückenden Stickstoffatom den 4-Morpholino-Rest bilden, bedeutet,
R¹² und R¹³ gleich oder verschieden sind und H, C₁₋₆-Alkyl oder C₃₋₈-Cycloalkyl oder R¹² und R¹³ zusammen eine Gruppe -(CH₂)₃-₈- als Teil eines Ringes bedeuten,
R¹⁴ H, OH, C₁₋₇-Alkyl, O-C₁₋₇-Alkyl, Phenyl, O-Aryl, CF₃, Cl oder F bedeutet, mit der Maßgabe, dass die beiden Reste R¹⁴ gleich oder verschieden sind.

Besonders bevorzugt kommen in den erfindungsgemäßen Verfahren substituierte 4-Dimethylamino-2-aryl-butan-2-ol-Verbindungen der allgemeinen Formel Ia zum Einsatz, in denen
R¹ C₁₋₃-Alkyl,
R² H oder C₁₋₃-Alkyl
R³ H oder C₁₋₃-Alkyl,
R⁴ H, OH, Cl, F oder OR⁸,
R⁵ H, OH, C₁₋₄-Alkyl, O-C₁₋₄-Alkyl, O-Benzyl, CHF₂, CF₃, Cl, F oder OR⁸,
und R⁶ H, OH, O-C₁₋₄-Alkyl O-Benzyl, CF₃, Cl, F oder OR⁸,
darstellt, mit der Maßgabe, dass zwei der Reste R⁴, R⁵ oder R⁶ H sind,
oder R⁴ und R⁵ zusammen eine Gruppe -CH=C(R⁹)-O- oder -CH=C(R⁹)-S-, jeweils als Teil eines Ringes bedeuten, mit der Maßgabe, dass R⁶ H ist, oder R⁵ und R⁶ zusammen eine Gruppe -CH=CH-C(OR¹⁰)=CH- als Teil eines Ringes bedeuten, mit der Maßgabe, dass R⁴ H ist,
R⁸ bis R¹⁰ die vorstehend genannte Bedeutung haben.

Ganz besonders bevorzugt kommen in den erfindungsgemäßen Verfahren substituierte 4-Dimethylamino-2-aryl-butan-2-ol-Verbindungen der allgemeinen Formel Ia zum Einsatz, worin
R¹ CH₃ oder C₃H₇ ist,
R² H, CH₃ oder CH₂CH₃ ist,
R³ H, CH₃ oder CH₂CH₃ ist,
R⁴ H oder OH ist,
R⁵ H, OH, OCH₃, CHF₂ oder OR⁸ ist,
R⁶ H, OH oder CF₃ ist,
mit der Maßgabe, dass zwei Reste R⁴, R⁵ oder R⁶ H sind, oder R⁴ und R⁵ zusammen eine Gruppe -CH=C(CH₃)-S- als Teil eines Ringes darstellen, mit der Maßgabe, dass R⁶ H ist, oder R⁵ und R⁶ zusammen -CH=CH-C(OH)=CH- als Teil eines Ringes bedeuten mit der Maßgabe, dass R⁴ H ist, R⁸ CO-C₆H₄-R¹¹ und R¹¹ -OC(O)-C₁₋₃-Alkyl in ortho-Stellung bedeutet,

In den erfindungsgemäßen Verfahren kommen am meisten bevorzugt 1-Dimethylamino-3-(3-methoxy-phenyl)-2-methyl-pentan-3-ol insbesondere in Form seiner isolierten Enatiomeren oder Diastereomeren oder in Form von Mischungen seiner Stereoisomerenzum Einsatz, wobei die erstgenannten Alkohol-Verbindungen nach dem erfindungsgemäßen Verfahren zur Herstellung von substituierten Dimethyl-(3-aryl-butyl)-amin-Verbindung der allgemeinen Formel III zu 3-(3-Methoxy-phenyl)-2-methyl-pentyl-dimethylamin und die letztgenannten Alkohol-Verbindungen zu 3-(3-Methoxy-phenyl)-2-methyl-pentyl-dimethylamin insbesondere in Form seiner isolierten Enatiomeren oder Diastereomeren oder in Form von Mischungen seiner Stereoisomeren umgesetzt werden.

Die substituierten 4-Dimethylamino-2-aryl-butan-2-ol-Verbindungen können nach üblichen, dem Fachmann bekannten Verfahren hergestellt werden, wie beispielsweise in EP 0 693 475 und EP 0 799 819 beschrieben, deren entsprechende Beschreibungen hiermit als Referenz eingeführt werden und als Teil der Offenbarung gelten.

Die durch Dehydratisierung substituierter 4-Dimethylamino-2-aryl-butan-2-ol-verbindungen erhaltenen substituierten Dimethyl-(3-aryl-but-3-enyl)-amin-verbindungen liegen, sofern gegeben, üblicherweise in Form eines Gemisches ihrer Stereoisomeren vor. Diese können nach üblichen, dem Fachmann bekannten Methoden, beispielsweise mit Hilfe chromatographischer Methoden voneinander getrennt werden.

Die Umsetzung der substituierten Dimethyl-(3-aryl-but-3-enyl)-amin-verbindungen der allgemeinen Formel II zu substituierten Dimethyl-(3-aryl-butyl)-amin-Verbindungen der allgemeinen Formel III führt ggf. ebenfalls zu einem Gemisch unterschiedlicher Stereoisomere, die nach üblichen, dem Fachmann bekannten Methoden voneinander getrennt werden können. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Der Fachmann versteht, daß der Einsatz unterschiedlich substituierter 4-Dimethylamino-2-aryl-butan-2-ol-Verbindungen der allgemeinen Formel I in den erfindungsgemäßen Verfahren zu entsprechend substituierten Dimethyl-(3-aryl-but-3-enyl)-Verbindungen der allgemeinen Formel II bzw. zu entsprechend substituierten Dimethyl-(3-aryl-butyl)-amin-Verbindungen der allgemeinen Formel III führt.

Die substituierten 4-Dimethylamino-2-aryl-butan-2-ol-Verbindungen der allgemeinen Formel I können ebenso wie die substituierten Dimethyl-(3-aryl-but-3-enyl)-aminverbindungen der allgemeinen Formel II in den erfindungsgemäßen Verfahren jeweils sowohl in Form ihrer Basen, ihrer Säuren als auch jeweils in Form ihrer Salze oder jeweils in Form entsprechender Solvate, vorzugsweise Hydrate, eingesetzt werden. Selbstverständlich können auch jeweils Mischungen aus zwei oder mehr der vorstehend genannten Verbindungen zum Einsatz kommen.

Sofern wenigstens eine substituierte 4-Dimethylamino-2-aryl-butan-2-ol-Verbindung der allgemeinen Formel I oder wenigstens eine substituierte Dimethyl-(3-aryl-but-3-enyl)-aminverbindung der allgemeinen Formel II in Form eines Salzes nach den erfindungsgemäßen Verfahren umgesetzt wird, kann dieses bevorzugt ausgewählt werden aus der Gruppe bestehend aus Chlorid, Bromid, Sulfat, Sulfonat, Phosphat, Tartrat, Teoclat, Embonat, Formiat, Acetat, Propionat, Benzoat, Oxalat, Succinat, Citrat, Diclofenacat, Naproxenat, Salycilat, Acetylsalicylat, Glutamat, Fumarat, Aspartat, Glutarat, Stearat, Butyrat, Malonat, Lactat, Mesylat, Saccharinat, Cyclamat und Acesulfamat, besonders bevorzugt aus der Gruppe Chlorid, Sulfat, Saccharinat, Teoclat, Embonat, Diclofenacat, Naproxenat, Ibuprofenat und Salicylat.

Üblicherweise liegen die Salze dabei in Form eines entsprechenden Säureadditionssalzes, z.B. als Hydrochlorid, vor.

Sofern die substituierten Dimethyl-(3-aryl-but-3-enyl)-amin-verbindungen der allgemeinen Formel II oder die substituierten Dimethyl-(3-aryl-butyl)-amin-Verbindungen der allgemeinen Formel III nach den erfindungsgemäßen Verfahren in Form ihrer Basen erhalten werden, können sie nach üblichen, dem Fachmann bekannten Verfahren in die entsprechenden Salze, vorzugsweise in eines der vorstehend aufgeführten Salze, übergeführt werden.

Heterogene Katalyse im Sinne der vorliegenden Erfindung bedeutet, daß die in den erfindungsgemäßen Verfahren zum Einsatz kommenden Katalysatoren jeweils im festen Aggregatzustand vorliegen.

Der Begriff Katalysator im Sinne der vorliegenden Erfindung umfaßt sowohl katalytisch aktive Materialien selbst als auch inerte Materialien, die mit einem katalytisch aktiven Material ausgerüstet sind. So kann das katalytisch aktive Material beispielsweise auf einen inerten Träger aufgebracht sein oder im Gemisch mit einem inerten Material vorliegen. Als inerter Träger oder inertes Material kommen beispielsweise Kohlenstoff und andere, dem Fachmann bekannten Materialien in Betracht.

Die in den erfindungsgemäßen Verfahren zum Einsatz kommenden substituierten 4-Dimethylamino-2-aryl-butan-2-ol-Komponenten bzw. die substituierten Dimethyl-(3-aryl-but-3-enyl)-amin-Komponenten liegen vorzugsweise in flüssiger Phase vor und werden dazu bevorzugt mit einem Reaktionsmedium gemischt oder darin gelöst, das unter den jeweiligen Reaktionsbedingungen flüssig ist.

Geeignete Reaktionsmedien sind beispielsweise Wasser oder organische Flüssigkeiten wie halogenierte organische Verbindungen, Alkohole oder Ketone, vorzugsweise Dichlormethan, Chloroform, Toluol oder Methanol, besonders bevorzugt Aceton oder insbesondere Ethanol. Selbstverständlich können auch Mischungen oder mehrphasige Systeme aus zwei oder mehr der vorstehend genannten Flüssigkeiten in den erfindungsgemäßen Verfahren eingesetzt werden. Möglich ist auch eine Umsetzung in überkritischem CO₂ als Lösungsmittel.

Die Dehydratisierung der substituierten 4-Dimethylamino-2-aryl-butan-2-ol-Verbindungen der allgemeinen Formel I erfolgt bevorzugt in Gegenwart wenigstens eines sauren Katalysators und/oder wenigstens eines basischen Katalysators, besonders bevorzugt in Gegenwart wenigstens eines sauren Katalysators. Es ist auch möglich, Katalysatoren einzusetzen, die sowohl sauer als auch basisch ausgerüstet sind.
Geeignete saure und/oder basische Katalysatoren können bevorzugt ausgewählt werden aus der Gruppe bestehend aus Ionenaustauscherharzen, Zeolithen, Heteropolysäuren, Phosphaten, Sulfaten und ggf. gemischten Metalloxiden.

Bevorzugt erfolgt die Dehydratisierung in Gegenwart wenigstens eines sauren Katalysators, der bevorzugt ausgewählt werden kann aus der Gruppe bestehend aus Ionenaustauscherharzen, Zeolithen, Heteropolysäuren, Phosphaten, Sulfaten und ggf. gemischten Metalloxiden.

Geignete Katalysatoren und deren Herstellung sind dem Fachmann an sich bekannt, beispielsweise aus Venuto, P.B., Microporous Mater., 1994, 2, 297; Hölderich, W.F., van Bekkum, H., Stud. Surf. Sci. Catal., 1991, 58, 631, Hölderich, W.F., Proceedings of the 10th International Congress on Catalysis, 1992, Budapest, Guczi, L. et al (Editors), "New Frontiers in Catalysis", 1993, Elsevier Science Publishers, Kozhenikov, I.V., Catal. Rev. Sci. Eng., 1995, 37, 311, Song, X., Sayari A., Catal. Rev. Sci. Eng., 1996, 38, 329. Die entsprechenden Literaturbeschreibungen werden hiermit als Referenz eingeführt und gelten als Teil der Offenbarung.

Für die Dehydratisierung eignen sich insbesondere solche Ionenaustauscherharze, die Sulfonsäuregruppen tragen.

Bevorzugt sind Ionenaustauscherharze auf Basis von Tetrafluorethylen/Perfluorvinylethyer-Copolymeren, ggf. in Form Ihrer Silica-Nanokomposite, wie sie beispielsweise in den Literaturveröffentlichungen von Olah et al. Synthesis, 1996, 513-531 und Harmer et al. Green Chemistry, 2000, 7-14 beschrieben werden, deren entsprechende Beschreibungen hiermit als Referenz eingeführt werden und als Teil der Offenbarung gelten.

Entsprechende Produkte sind am Markt beispielsweise unter der Bezeichnung Nafion^{®} käuflich erhältlich und können in dieser Form auch in den erfindungsgemäßen Verfahren eingesetzt werden.

Weiterhin bevorzugt sind Ionenaustauscherharze auf Basis von Styrol/Divinylbenzol-Copolymeren, die nach üblichen, dem Fachmann bekannten Verfahren hergestellt werden können.

Besonders bevorzugt kommen für die Dehydratisierung Sulfonsäure-Gruppen tragende Ionenaustauscherharze auf Basis von Styrol/Divinylbenzol-Copolymeren in Betracht, wie sie beispielsweise unter der Bezeichnung Amberlyst^{®} von der Firma Rohm & Haas am Markt geführt werden und als solche auch in den erfindungsgemäßen Verfahren eingesetzt werden können. Diese Ionenaustauscherharze zeichnen sich insbesondere durch ihre Stabilität gegenüber Wasser und Alkoholen, auch bei erhöhten Temperaturen, wie z.B. 130 bis 160 °C aus.

Der Vernetzungsgrad und die Struktur dieser Ionenaustauscherharze kann variieren. Beispielsweise seien makroporöse Ionenaustauscherharze mit heterogener Porendurchmesserverteilung, isoporöse Ionenaustauscherharze mit nahezu einheitlicher Porendurchmesserverteilung oder gelartige, keine oder nahezu keine Poren aufweisenden Ionenaustauscherharze genannt. Für die heterogene Katalyse in flüssiger Phase sind insbesondere die makroporösen Harze besonders vorteilhaft anwendbar.

Besonders geeignete makroporöse Harze mit einem mittleren Porendurchmesser von 20 bis 30 nm und einer Mindeskonzentration an aktiven Gruppen von 4,70 bis 5,45 Äquivalenten pro kg Harz sind unter den Bezeichnungen Amberlyst^{®} 15, Amberlyst^{®} 35 und Amberlyst^{®} 36 am Markt käuflich erhältlich und können entsprechend auch in den erfindungsgemäßen Verfahren eingesetzt werden.

Ebenfalls bevorzugt kann die Dehydratisierung in Gegenwart eines sauren Katalysators auf Basis von Metalloxiden wie z.B. SiO₂, Al₂O₃, TiO₂, Nb₂O₅, B₂O₃ oder auf Basis von gemischten Metalloxiden wie z.B. Al₂O₃/SiO₂ oder Al₂O₃/B₂O₃ erfolgen.

Die jeweiligen Reaktionssparameter der erfindungsgemäßen Verfahren, wie beispielsweise Druck, Temperatur oder Umsetzungsdauer können über einen weiten Bereich variieren.

Vorzugsweise beträgt die Temperatur während dieser Reaktionen jeweils 20-250°C besonders bevorzugt 50-180°C und ganz besonders bevorzugt 100-160° C.

Beide Reaktionen können bei vermindertem Druck, bei Normaldruck und auch bei erhöhtem Druck, vorzugsweise im Bereich von 0,01 bis 300 bar, durchgeführt werden. Besonders bevorzugt ist die Durchführung unter Druck in einem Bereiche von 2 bis 10 bar, insbesondere 4 bis 8 bar.

Die jeweilige Reaktionsdauer kann in Abhängigkeit verschiedener Parametern, wie z.B. Temperatur, Druck, Art der umzusetzenden Verbindung oder der Beschaffenheit des Katalysators, variieren und kann für das jeweilige Verfahren vom Fachmann durch Vorversuche ermittelt werden.

Das Verhältnis von Katalysator und umzusetzender Verbindung liegt bevorzugt im Bereich zwischen 1:200 bis 1:1, insbesondere 1:4 bis 1:2.

Nach der Dehydratisierung kann der Katalysator auf einfache Art und Weise, vorzugsweise durch Filtration, von der Reaktionsmischung abgetrennt werden. Die jeweilige, als Zwischen- oder Endprodukt erhaltene substituierte Dimethyl-(3-aryl-but-3-enyl)-amin-Verbindung der allgemeinen Formel II kann nach herkömmlichen, dem Fachmann bekannten Methoden isoliert und/oder gereinigt werden.

Die weitere Umsetzung der als Zwischenprodukt erhaltenen Dimethyl-(3-aryl-but-3-enyl)-amin-Verbindungen der allgemeinen Formel II kann bevorzugt ebenfalls unter heterogener Katalyse erfolgen.

In einer bevorzugten Ausführungsform des erfindungsgemäßen Verfahrens erfolgt die Umsetzung der als Zwischenprodukt erhaltenen substituierten Dimethyl-(3-aryl-but-3-enyl)-amin-Verbindung der allgemeinen Formel II durch eine heterogen katalysierte Hydrierung mit Wasserstoff. Der Wasserstoff liegt dabei vorzugsweise gasförmig vor, kann aber auch zumindest teilweise in einer flüssigen Phase gelöst sein.

Vorzugsweise erfolgt die Umsetzung der als Zwischenprodukt erhaltenen Dimethyl-(3-aryl-but-3-enyl)-amin-Verbindungen der allgemeinen Formel II unter heterogener Katalyse in Gegenwart wenigstens eines Katalysators, der ein oder mehrere Übergangsmetalle aufweist, und ggf. in Gegenwart wenigstens einer der vorstehend für die Dehydratisierung zum Einsatz kommenden Katalysatoren. Alternativ kann diese Umsetzung auch in Gegenwart wenigstens einer der nachstehend beschriebenen polyfunktionalisierten, vorzugsweise bifunktionaliserten, Katalysatoren erfolgen.

In einer bevorzugten Ausführungsform des erfindungsgemäßen Verfahrens zur Herstellung substituierter Dimethyl-(3-aryl-butyl)-amin-Verbindungen der allgemeinen Formel III erfolgt die Dehydratisierung zum Zwischenprodukt und dessen Umsetzung ohne Reinigung und/oder Isolierung zum Endprodukt in Gegenwart wenigstens eines polyfunktionalisierten, vorzugsweise bifunktionalisierten, Katalysators.
Unter bi- bzw. polyfunktionalisierten Katalysatoren werden erfindungsgemäß solche Katalysatoren verstanden, die zwei oder mehr verschiedene Funktionalitäten aufweisen und daher zwei oder mehr verschiedene Reaktionen, vorzugsweise zumindest die Dehydratisierung und die nachfolgende Umsetzung des so erhaltenen Zwischenproduktes, beschleunigen können.

Bevorzugt sind bifunktionalisierte Katalysatoren, die sauer und/oder basisch, vorzugsweise sauer, sind und wenigstens ein Übergangsmetall aufweisen.

Besonders bevorzugt leiten sich diese, bifunktionalisierten Katalysatoren von einem der vorstehend für die Dehydratisierung genannten, vorzugsweise sauren, Katalysatoren ab.

Sofern einer der in den erfindungsgemäßen Verfahren zum Einsatz kommenden Katalysatoren eines oder mehrere Übergangsmetalle aufweist, können diese bevorzugt ausgewählt werden aus der Gruppe bestehend aus Cu, Ag, Au, Zn, Cd, Hg, V, Nb, Ta, Cr, Mo, W, Fe, Ru, Os, Co, Rh, Ir, Ni, Pd, Pt, besonders bevorzugt aus der Gruppe bestehend aus Ru, Rh, Pd, Os, Ir und Pt und ganz besonders bevorzugt aus der Gruppe bestehend aus Pd, Ru, Pt und Ir. Am meisten bevorzugt ist Palladium.

Die entsprechenden Katalysatoren können bevorzugt eines oder mehrere der vorstehend genannten Übergangsmetalle in der gleichen oder verschiedenen Oxidationsstufen aufweisen. Gegebenenfalls ist es auch bevorzugt, wenn die entsprechenden Katalysatoren eines oder mehrere der vorstehend genannten Übergangsmetalle in zwei oder mehr unterschiedlichen Oxidationsstufen aufweisen.

Die Herstellung von Übergangsmetall-dotierten Katalysatoren kann nach üblichen, dem Fachmann bekannten Verfahren erfolgen.

Die Herstellung entsprechend bifunktionalisierter Katalysatoren kann ebenfalls nach üblichen, dem Fachmann bekannten Methoden erfolgen, beispielsweise in dem ein Ionenaustauscher partiell mit Übergangsmetallionen belegt wird oder durch Imprägnierung mit Übergangsmetallsalz-enthaltenden Lösungen, wie z.B. in Sachtler et al., Advances in Catalysis, 1993, 39, 129 beschrieben. Die entsprechende Literaturbeschreibung wird hiermit als Referenz eingeführt und gilt als Teil der Offenbarung.

Des weiteren wurde gefunden, daß die Hydrierung wenigstens einer substituierten Dimethyl-(3-aryl-but-3-enyl)-amin-Verbindung der allgemeinen Formel II zu wenigstens einer substituierten Dimethyl-(3-aryl-butyl)-amin-Verbindung der allgemeinen Formel III mit Wasserstoff besonders vorteilhaft verläuft, wenn sie in Gegenwart eines Katalysatorgemisches oder in Gegenwart eines polyfunktionalisierten, vorzugsweise bifunktionalisierten Katalysators erfolgt.

Ein weiterer Gegenstand der vorliegenden Erfindung ist daher ein Verfahren zur Herstellung wenigstens einer substituierten Dimethyl-(3-aryl-butyl)-amin-verbindung der allgemeinen Formel III, 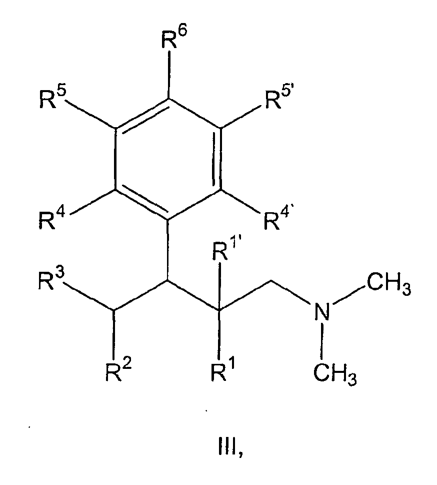 worin die Reste R¹, R¹', R², R³, R⁴, R^{4'}, R⁵, R^{5'} und R⁶ die vorstehend genannte Bedeutung haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, **dadurch gekennzeichnet, daß** wenigstens eine substituierte Dimethyl-(3-aryl-but-3-enyl)-amin-Verbindung der allgemeinen Formel II 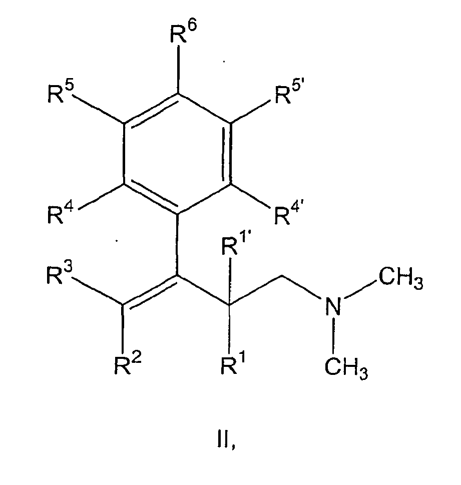 worin die Reste R¹, R¹', R², R³, R⁴, R^{4'}, R⁵, R^{5'} und R⁶ die vorstehend genannte Bedeutung haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, durch Hydrierung mit Wasserstoff unter heterogener Katalyse in Gegenwart eines Gemisches aus wenigstens einem der vorstehend für die Dehydratisierung genannten Katalysatoren und wenigstens einem Katalysator, der ein oder mehrere Übergangsmetalle aufweist, oder in Gegenwart wenigstens einer der vorstehend genannten bifunktionalisierten Katalysatoren zu einer Verbindung der allgemeinen Formel III als Endprodukt umgesetzt wird.

Das erfindungsgemäße Verfahren ist besonders geeignet, um aus (-)-(2S,3S)-1-Dimethylamino-3-(3-methoxy-phenyl)-2-methyl-pentan-3-ol ein Gemisch aus (-)-(2R,3R)-[3-(3-methoxy-phenyl)-2-methyl-pentyl]-dimethylamin und (-)-(2R,3S)-[3-(3-methoxy-phenyl)-2-methyl-pentyl]-dimethylamin herzustellen.
Die jeweiligen Reaktionssparameter der erfindungsgemäßen Verfahren, wie beispielsweise Druck, Temperatur oder Umsetzungsdauer können sowohl bei der Dehydratisierung der substituierten 4-Dimethylamino-2-aryl-butan-2-ol-Verbindungen der allgemeinen Formel I als auch bei der Umsetzung der substituierten Dimethyl-(3-aryl-but-3-enyl)-Verbindungen der allgemeinen Formel II über einen weiten Bereich variieren.

Vorzugsweise beträgt die Temperatur während dieser Reaktionen jeweils 20-250°C, besonders bevorzugt 50-180°C und ganz besonders bevorzugt 100-160° C.

Beide Reaktionen können bei vermindertem Druck, bei Normaldruck und auch bei erhöhtem Druck, vorzugsweise im Bereich von 0,01 bis 300 bar, durchgeführt werden. Besonders bevorzugt ist die Durchführung unter Druck in einem Bereich von 2 bis 10 bar, insbesondere 4 bis 10 bar.

Die jeweilige Reaktionsdauer kann in Abhängigkeit verschiedener Parametern, wie z.B. Temperatur, Druck, Art der umzusetzenden Verbindung oder der Beschaffenheit des Katalysators, variieren und kann für das jeweilige Verfahren vom Fachmann durch Vorversuche ermittelt werden.

Auch eine kontinuierliche Probenentnahme zur Kontrolle der jeweiligen Reaktion zum Beispiel mittels gaschromatographischer Methoden ist möglich, ggf. in Kombination mit der Regelung der entsprechenden Verfahrensparameter, ist möglich.

Die Menge des/der eingesetzten Katalysators/Katalysatoren hängt von verschiedenen Faktoren ab, wie beispielsweise von Verhältnis der katalytischen aktiven Komponente zu ggf. vorhandenem inertem Material oder von der Oberflächenbeschaffenheit des Katalysators. Die für die jeweilige Reaktion optimale Menge des jeweiligen Katalysators kann vom Fachmann durch Vorversuche bestimmt werden.

Für die Dehydratisierung und Hydrierung in Gegenwart von bifunktionalisierten lo1nenaustauscherharzen auf Basis von Styrol/Divinylbenzol-Copolymeren, die Sulfonsäure-Gruppen tragen und mit 0,1 bis 10 Gew.-%, bevorzugt mit 0,3 bis 3 Gew.-%, besonders bevorzugt mit 0,5 bis 2 Gew.-%, jeweils bezogen auf das Gesamtgewicht des Katalysators, Palladium ausgerüstet sind, können der Katalysator und die umzusetzende Verbindung bevorzugt im Verhältnis von 1:200 bis 1:1, besonders bevorzugt von 1:4 bis 1:2 eingesetzt werden.

Die erfindungsgemäßen Verfahren können jeweils diskontinuierlich (batchweise) oder kontinuierlich durchgeführt werden, wobei die diskontinuierliche Verfahrensführung bevorzugt ist.

Als Reaktor kommt für die diskontinuierliche Verfahrensführung beispielsweise ein slurry-Reaktor, für die kontinuierliche Verfahrensführung ein Festbettreaktor oder Schlaufenreaktor in Betracht.

Die Dehydratisierung substituierter 4-Dimethylamino-2-aryl-butan-2-ol-Verbindungen der allgemeinen Formel I und ggf. die nachfolgende Umsetzung der so erhaltenen substituierten Dimethyl-(3-aryl-but-3-enyl)-amin-Verbindungen der allgemeinen Formel II gelingt nach den erfindungsgemäßen Verfahren in guter Ausbeute mit sehr guten Reinheiten.

Im Vergleich zu den herkömmlichen Herstellungsverfahren entfällt zudem eine Chlorierung des Alkohols mit aggressiven Reagentien bzw. die Aufarbeitung und Entsorgung vergleichsweise großer Mengen an Säure.

In dem die Dehydratisierung und die anschließende Umsetzung der dehydratisierten Verbindung zur Herstellung substituierter Dimethyl-(3-aryl-butyl)-aminverbindungen der allgemeinen Formel III, vorzugsweise durch Hydrierung, in einem gemeinsamen Verfahrensschritt durchgeführt werden, kann die Umweltbilanz sowie die Dauer dieses Verfahrens und somit dessen Wirtschaftlichkeit darüber hinaus noch weiter verbessert werden.

Die erfindungsgemäß eingesetzten festen Katalysatoren können zudem auf einfache Art und Weise von der jeweiligen Reaktionsmischung abgetrennt, ggf. regeneriert und erneut eingesetzt werden.

Im folgenden wird die Erfindung anhand von Beispielen erläutert. Diese Erläuterungen sind lediglich beispielhaft und schränken den allgemeinen Erfindungsgedanken nicht ein.

### Beispiele:

### Beispiel 1

### Herstellung des bifunktionellen Katalysators:

**1a)**
   In einem Rundkolben wurden 2 g eines Sulfonsäure-Gruppe tragenden Ionenaustauscherharzes auf Basis eines Styrol/Divinylbenzol-Copolymeren mit einem Divinylbenzol-Gehalt von 20 Gew.-%, bezogen auf das Gesamtgewicht des Harzes, einem mittleren Porendurchmesser von 25 nm und einer Mindestkonzentration an aktiven Gruppen von 4,70 Äquivalenten pro kg Harz (Amberlyst^{®} 15, Fluka, Schweiz) in 20 ml Wasser suspendiert. Anschließend wurden 0,3 ml einer Palladium-Tetramindinitrathydrat-Lösung mit einem Palladium-Gehalt von 69,5 mg/ml zugegeben und für 24 Stunden bei einer Temperatur von 80°C gerührt. Zur Herstellung der Palladium(II)tetraminnitrathydrat-Komplex-Lösung wurden 10 g Palladium(II)nitrat Dihydrat (Fluka) in 400 g Ammoniaklösung (25 Gew.-% in Wasser) gegeben und die so erhaltene Mischung für drei Tage bei 50 °C gerührt. Anschließend wurde der unerwünschte Feststoff durch Filtration abgetrennt und der Palladiumgehalt mittels ICP-AES bestimmt, wie in R. Meiers, Dissertation RWTH-Aachen, Shaker-Verlag (1998) beschrieben. Die entsprechende Beschreibung wird hiermit als Referenz eingeführt und gilt als Teil der Offenbarung. Anschließend wurde der so erhaltene Katalysator filtriert, mit Wasser gewaschen und im Feinvakuum für 3-4 Stunden bei 120°C getrocknet. Das fertige Produkt hat einen Pd-Gehalt von 1 Gew.-%.
**1b)**
   In einem Rundkolben wurden 2 g eines Sulfonsäure-Gruppe tragenden Ionenaustauscherharzes auf Basis eines Styrol/Divinylbenzol-Copolymeren mit einem Divinylbenzol-Gehalt von 12 Gew.-%, bezogen auf das Gesamtgewicht des Harzes, einem mittleren Porendurchmesser von 20 nm und einer Mindestkonzentration an aktiven Gruppen von 5,45 Äquivalenten pro kg Harz (Amberlyst^{®} 36, Fluka, Schweiz) in 20 ml Wasser suspendiert. Anschließend wurden 0,3 ml einer gemäß Beispiel 1a hergestellten Palladium-Tetramindinitrathydrat-Lösung mit einem Palladium-Gehalt von 69,5 mg/ml zugegeben und für 24 Stunden bei einer Temperatur von 80°C gerührt. Anschließend wurde der so erhaltene Katalysator filtriert, mit Wasser gewaschen und im Feinvakuum für 3-4 h bei 120°C getrocknet. Das fertige Produkt hat einen Pd-Gehalt von 1 Gew.-%.

### Beispiel 2:

### Synthese von (Z;E)-(S)-[3-(3-methoxy-phenyl)-2-methyl-pent-3-enyl]-dimethyl-amin; hydrochlorid (2) aus (-)-(2S,3S)-1-Dimethylamino-3-(3-methoxy-phenyl)-2-methyl-pentan-3-ol; hydrochlorid (1):

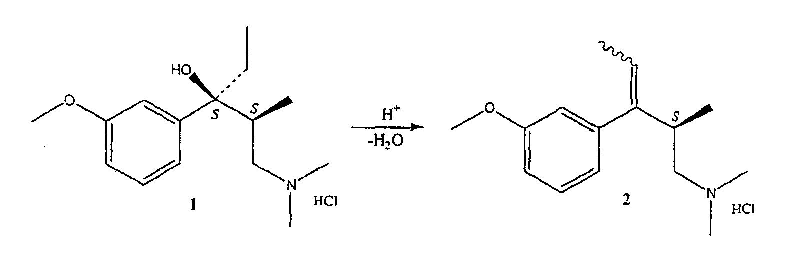

In einem 75 ml Edelstahlautoklaven wurden 1,0 g (3,4 mmol) (-)-(2*S*,3*S*)-1-Dimethylamino-3-(3-methoxy-phenyl)-2-methyl-pentan-3-ol; hydrochlorid (1) und 0,5 g eines Sulfonsäure-Gruppe tragenden Ionenaustauscherharzes auf Basis eines Styrol/Divinylbenzol-Copolymeren mit einem Divinylbenzol-Gehalt von 20 Gew.-%, bezogen auf das Gesamtgewicht des Harzes, einem mittleren Porendurchmesser von 25 nm und einer Mindestkonzentration an aktiven Gruppen von 4,70 Äquivalenten pro kg Harz (Amberlyst^{®} 15, Fluka, Schweiz) vorgelegt. Nach Zugabe von 15 ml frisch destilliertem Ethanol wurde die Reaktionsmischung bei 150°C für 4 Stunden im geschlossenem System (bei einem Gesamtdruck von bis zu 8 bar) gerührt. Nach dem Abkühlen auf Raumtemperatur (ca. 20-25°C) wurde der Katalysator abfiltriert. Von dem so erhaltenen Filtrat wurde eine Probe entnommen und mit einer 50 m SE-54-Säule der Firma Chrompack gaschromatographisch untersucht.

Es wurden > 98 % an (-)-(2*S*,3*S*)-1-Dimethylamino-3-(3-methoxy-phenyl)-2-methylpentan-3-ol; hydrochlorid (1) umgesetzt.

Die Ausbeute an (*Z*;*E*)-(S)-[3-(3-methoxy-phenyl)-2-methyl-pent-3-enyl]-dimethylamin; hydrochlorid (2) betrug 92-95 % bei einem Verhältnis Z : E von 70 : 30.

### Beispiel 3:

### Synthese von (-)-(2R,3R)-[3-(3-methoxy-phenyl)-2-methyl-pentyl]-dimethylamin; hydro-chlorid (3a) und (-)-(2R,3S)-[3-(3-methoxy-phenyl)-2-methyl-pentyl]-dimethyl-amin; hydro-chlorid (3b) aus (Z)-(S)-[3-(3-methoxy-phenyl)-2-methyl-pent-3-enyl]-dimethyl-amin; hydrochlorid (2):

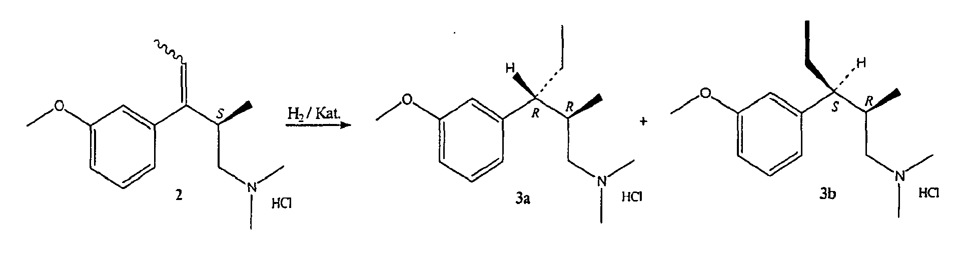

In einem 75 ml Edelstahlautoklaven wurden 1,0 g (3,7 mmol) (*Z*)-(*S*)-[3-(3-methoxyphenyl)-2-methyl-pent-3-enyl]-dimethyl-amin; hydrochlorid (2) und 0,5 g des gemäß Beispiel 1a erhaltenen Katalysators vorgelegt. Der Autoklav wurde im Feinvakuum evakuiert und anschließend mit Argon begast. Unter Argon-Atmosphäre wurden 15 ml frisch destilliertes Ethanol hinzugefügt. Anschließend wurden bei Raumtemperatur 4 bar H₂ aufgepresst und die Reaktionsmischung bei 150°C für 4 h (Gesamtdruck bei 150°C bis 12 bar) gerührt. Nach Abkühlen auf Raumtemperatur wurde der überschüssige Wasserstoff abgelassen und der Katalysator abfiltriert. Vom Filtrat wurde eine Probe entnommen und mit einer 50 m SE-54-Säule der Firma Chrompack gaschromatographisch untersucht.

Es wurden > 98 % (*Z*)-(*S*)-[3-(3-methoxy-phenyl)-2-methyl-pent-3-enyl]-dimethylamin; hydro-chlorid umgesetzt.

Die Ausbeute an [3-(3-methoxy-phenyl)-2-methyl-pentyl]-dimethyl-amin; hydrochlorid (3) betrug > 96% mit einem Verhältnis Enantiomer (-)-(2R,3R) zum Diastereomer (-)-(2*R*,3*S*) von 76 : 24. Das (-)-(2R,3R)-Enantiomer wird bevorzugt als Wirkstoff in Arzneimitteln eingesetzt.

### Beispiel 4:

### Direkte Synthese von (-)-(2R,3R)-[3-(3-methoxy-phenyl)-2-methyl-pentyl]-dimethyl-amin; hydrochlorid aus (-)-(2S,3S)-1-Dimethylamino-3-(3-methoxyphenyl)-2-methyl-pentan-3-ol; hydrochlorid :

**4a**
   In einem 75 ml Edelstahlautoklaven wurden 1,0 g (3,4 mmol) (-)-(2*S*,3*S*)-1-Dimethylamino-3-(3-methoxy-phenyl)-2-methyl-pentan-3-ol; hydrochlorid und 0,5 g des gemäß Beispiel 1a erhaltenen Katalysators vorgelegt. Der Autoklav wurde im Feinvakuum evakuiert und dann mit Argon begast. Unter Argon-Atmosphäre wurden 15 ml frisch destilliertes Ethanol hinzugefügt. Anschließend wurden bei Raumtemperatur 4 bar H₂ aufgepresst und die Reaktionsmischung bei 150°C für 4 h (Gesamtdruck bei 150°C bis 12 bar) gerührt. Nach Abkühlen auf Raumtemperatur wurde der überschüssige Wasserstoff abgelassen und der Katalysator abfiltriert. Vom Filtrat wurde eine Probe entnommen und mit einer 50 m SE-54-Säule der Firma Chrompack gaschromatographisch untersucht.
   Zum Isolieren der kristallinen Produktmischung wurde zunächst das Ethanol am Rotationsverdampfer entfernt und anschließend das so erhaltene Rohprodukt aus Heptan : Tetrahydrofuran in einem Verhältnis von 1 : 1 (Volumen/Volumen) umkristallisiert. Es wurden farblose Kristalle erhalten, die erneut mit Hilfe der Gaschromatographie analysiert wurden.
   Der Umsatz an (*Z*)-(*S*)-[3-(3-methoxy-phenyl)-2-methyl-pent-3-enyl]-dimethyl-amin; hydrochlorid 2 betrug > 98%.
   Die Ausbeute an [3-(3-methoxy-phenyl)-2-methyl-pentyl]-dimethyl-amin; hydrochlorid (3) betrug = 91 % (der Theorie) mit einem Diastereomerenverhältnis der (R,R) konfigurierten Verbindung, die bevorzugt als pharmazeutischer Wirkstoff in einem Arzneimittel eingesetzt wird, zu der (S,R) konfigurierten Verbindung von 64 : 36.
**4b)**
   Die Herstellung erfolgte analog zu Beispiel 4a), wobei anstelle des gemäß Beispiel 1a) erhaltenen Katalysators der gemäß Beispiel 1b) erhaltene Katalysator eingesetzt wurde.

Es wurden > 95 % an (*Z*)-(*S*)-[3-(3-methoxy-phenyl)-2-methyl-pent-3-enyl]-dimethylamin; hydrochlorid umgesetzt.

Die Ausbeute an [3-(3-methoxy-phenyl)-2-methyl-pentyl]-dimethyl-amin; hydrochlorid betrug 90% mit einem Verhältnis der (*R*,*R*) konfigurierten Verbindung zum Diastereomer (*S*,*R*) von 61 : 39.

## Patentansprüche

1. Verfahren zur Dehydratisierung wenigstens einer substituierten 4-Dimethylamino-2-aryl-butan-2-ol-Verbindung der allgemeinen Formel I, 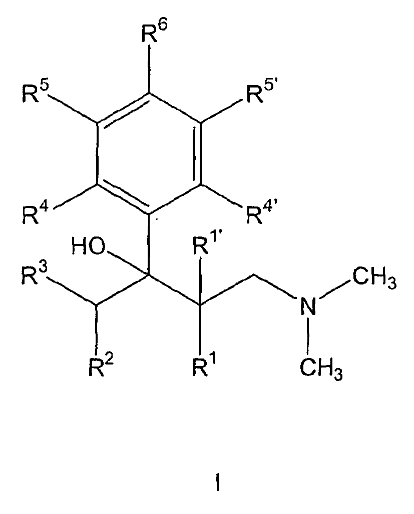 worin
R¹ H oder C₁₋₅-Alkyl bedeutet,
R^{1'} H oder C₁₋₅-Alkyl bedeutet,
R² H oder C₁₋₅-Alkyl bedeutet,
R³ H oder C₁₋₅-Alkyl bedeutet,
R⁴, R^{4'}, R⁵, R^{5'}, R⁶, gleich oder verschieden, jeweils für H, OH, C₁₋₄-Alkyl, OC₁₋₄-Alkyl teil- oder perfluoriertes C₁₋₄-Alkyl, teil- oder perfluoriertes O-C₁₋₄-Alkyl, O-(CH₂)ₙ-Phenyl mit n = 1, 2 oder 3, F, Cl oder OR⁸ stehen,
oder zwei benachbarte Reste R⁴ und R⁵, R⁵ und R⁶, R⁶ und R^{5'} oder R^{5'} und R^{4'} für eine Gruppe -OCH=CHO-, -CH=C(R⁹)-O-, -CH=C(R⁹)-S- oder -CH=CH-C(OR¹⁰)=CH- als Teil eines Ringes stehen, mit der Maßgabe, daß die jeweils anderen Reste R⁶, R^{5'} und R^{4'}, R⁴, R^{5'} und R^{6'}, R⁴, R⁵ und R^{4'} bzw. R⁴, R⁵ und R⁶ die vorstehend genannte Bedeutung haben,
R⁸ CO-C₁₋₅-Alkyl, PO(O-C₁₋₄-Alkyl)₂, CO-C₆H₄-R¹¹, CO(O-C₁₋₅-Alkyl), CO-CHR¹²-NHR¹³, CO-NH-C₆H₃-(R¹⁴)₂ oder eine unsubstituierte oder wenigstens einfach substituierte Pyridyl-, Thienyl-, Thiazoyl- oder Phenylgruppe bedeutet,
R⁹ H oder C₁₋₄-Alkyl bedeutet,
R¹⁰ H oder C₁₋₃-Alkyl bedeutet,
R¹¹ OC(O)-C₁₋₃-Alkyl in ortho-Stellung oder -CH₂-N-(R₁₅)₂ in meta- oder para-Stellung, wobei R¹⁵ jeweils C₁₋₄-Alkyl oder beide Reste R¹⁵ zusammen mit dem verbrückenden Stickstoffatom einen 4-Morpholino-Rest bilden,
R¹² und R¹³, gleich oder verschieden, jeweils für H, C₁₋₆-Alkyl oder C₃₋₈-Cycloalkyl stehen,
oder R¹² und R¹³ zusammen -(CH₂)₃₋₈ bedeuten,
R¹⁴ H, OH, C₁₋₇-Alkyl, teil- oder perfluoriertes C₁₋₇-Alkyl, OC₁₋₇-Alkyl, Phenyl, O-Aryl, F, Cl mit der Maßgabe, daß die Reste R¹⁴ gleich oder verschieden sind,
jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salze, oder jeweils in Form eines Solvates,
**dadurch gekennzeichnet, daß** sie unter heterogener Katalyse zu einer substituierten Dimethyl-(3-aryl-but-3-enyl)-amin-Verbindung der allgemeinen Formel II, 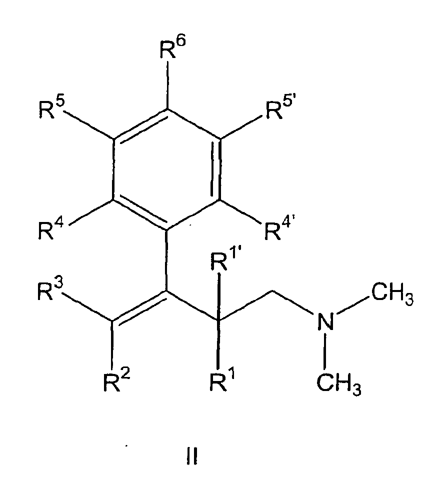 worin R¹, R¹', R², R³, R⁴, R^{4'}, R⁵, R^{5'} und R⁶ die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, dehydratisiert wird.

2. Verfahren zur Herstellung wenigstens einer substituierten Dimethyl-(3-arylbutyl)-amin-verbindung der allgemeinen Formel III, 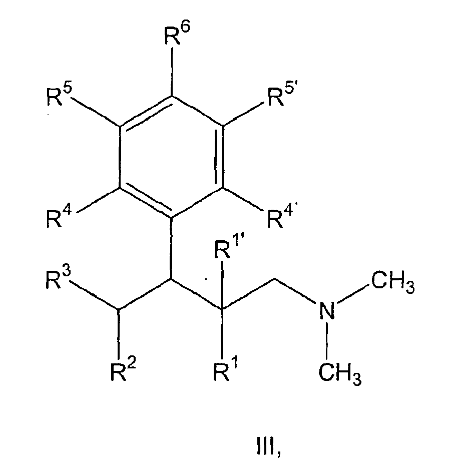 worin die Reste R¹, R¹', R², R³, R⁴, R^{4'}, R⁵, R^{5'} und R⁶ die Bedeutung gemäß Anspruch 1 haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, **dadurch gekennzeichnet, daß** wenigstens eine substituierte 4-Dimethylamino-2-aryl-butan-2-ol-Verbindung der allgemeinen Formel I 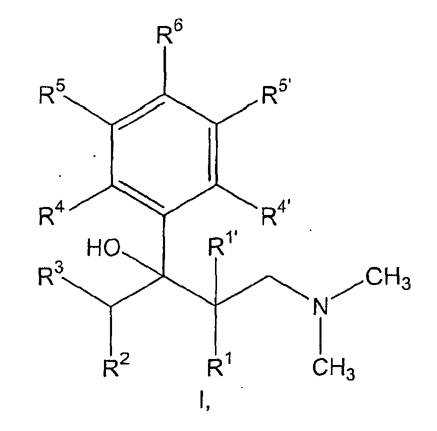 worin die Reste R¹, R¹', R², R³, R⁴, R^{4'}, R⁵, R^{5'} und R⁶ die Bedeutung gemäß Anspruch 1 haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salze, oder jeweils in Form eines Solvates, unter heterogener Katalyse zu einer substituierten Dimethyl-(3-aryl-but-3-enyl)-amin-Verbindung der allgemeinen Formel II, 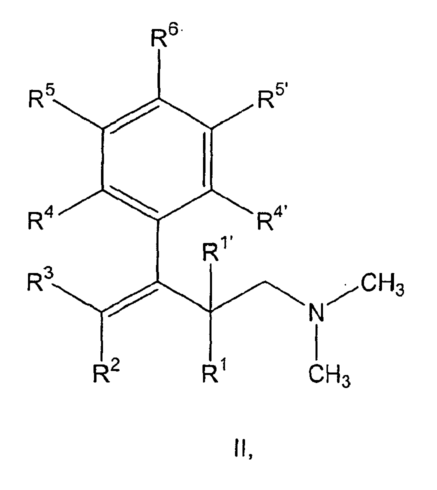 worin die Reste R¹, R¹', R², R³, R⁴, R^{4'}, R⁵, R^{5'} und R⁶ die Bedeutung gemäß Anspruch 1 haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, als Zwischenprodukt dehydratisiert wird, dieses Zwischenprodukt ggf. isoliert, ggf. gereinigt und zu einer Dimethyl-(3-aryl-butyl)-amin-Verbindung der allgemeinen Formel III als Endprodukt umgesetzt wird.

3. Verfahren gemäß Anspruch 2, **dadurch gekennzeichnet, daß** die Umsetzung zur Verbindung der allgemeinen Formel III unter heterogener Katalyse erfolgt.

4. Verfahren gemäß Anspruch 2 oder 3, **dadurch gekennzeichnet, daß** die Umsetzung zur Verbindung der allgemeinen Formel III durch Hydrierung mit Wasserstoff erfolgt.

5. Verfahren gemäß einem der Ansprüche 1 bis 4, **dadurch gekennzeichnet, daß** die Dehydratisierung in Gegenwart wenigstens eines sauren Katalysators und/oder wenigstens eines basischen Katalysators, vorzugsweise in Gegenwart wenigstens eines sauren Katalysators, erfolgt.

6. Verfahren gemäß Anspruch 5, **dadurch gekennzeichnet, daß** der/die saure(n) Katalysator(en) ausgewählt ist/sind aus der Gruppe bestehend aus Ionenaustauscherharzen, Zeolithen, Heteropolysäuren, Phosphaten, Sulfaten und ggf. gemischten Metalloxiden.

7. Verfahren gemäß Anspruch 6, **dadurch gekennzeichnet, daß** das Ionenaustauscherharz Sulfonsäuregruppen aufweist.

8. Verfahren gemäß Anspruch 6 oder 7, **dadurch gekennzeichnet, daß** das Ionenaustauscherharz auf wenigstens einem Tetrafluorethylen/Perfluorvinylether-Copolymeren basiert.

9. Verfahren gemäß Anspruch 6 oder 7, **dadurch gekennzeichnet, daß** das Ionenaustauscherharz auf wenigstens einem Styrol/Divinylbenzol-Copolymeren basiert.

10. Verfahren gemäß Anspruch 6, **dadurch gekennzeichnet, daß** ein Metalloxid ausgewählt aus der Gruppe bestehend aus SiO₂, Al₂O₃, TiO₂, Nb₂O₅ und B₂O₃ und/oder Al₂O₃/SiO₂ und/oder Al₂O₃/B₂O₃ als gemischtes Metalloxid vorliegt.

11. Verfahren gemäß einem der Ansprüche 3 bis 10, **dadurch gekennzeichnet, daß** die Umsetzung zur Verbindung der allgemeinen Formel III in Gegenwart wenigstens eines Katalysators erfolgt, der ein oder mehrere Übergangsmetalle aufweist, und gegebenenfalls in Gegenwart wenigstens eines Katalysators gemäß den Ansprüchen 5 bis 10.

12. Verfahren gemäß einem der Ansprüche 3 bis 10, **dadurch gekennzeichnet, daß** die Umsetzung zum Endprodukt in Gegenwart wenigstens eines polyfunktionalisierten, vorzugsweise bifunktionalisierten, Katalysators erfolgt.

13. Verfahren gemäß Anspruch 3 oder 4, **dadurch gekennzeichnet, daß** die Dehydratisierung zum Zwischenprodukt und dessen Umsetzung ohne Reinigung und/oder Isolierung zum Endprodukt in Gegenwart wenigstens eines polyfunktionalisierten, vorzugsweise bifunktionalisierten, Katalysators, erfolgt.

14. Verfahren gemäß Anspruch 12 oder 13, **dadurch gekennzeichnet, daß** als bifunktionalisierte Katalysator ein Katalysator zum Einsatz kommt, der sauer und/oder basisch, vorzugsweise sauer, ist und ein oder mehrere Übergangsmetalle aufweist.

15. Verfahren gemäß Anspruch 14, **dadurch gekennzeichnet, daß** als saurer Katalysatormidestens ein Ionenaustauscherharz verwendet wird, welches ein oder mehrere Übergangsmetalle aufweist.

16. Verfahren gemäß Anspruch 15, **dadurch gekennzeichnet, daß** das Ionenaustauscherharz Sulfonsäuregruppen aufweist.

17. Verfahren gemäß Anspruch 15 oder 16, **dadurch gekennzeichnet, daß** das Ionenaustauscherharz auf wenigstens einem Tetrafluorethylen/Perfluorvinylether-Copolymeren basiert.

18. Verfahren gemäß Anspruch 15 oder 16, **dadurch gekennzeichnet, daß** das Ionenaustauscherharz auf wenigstens einem Styrol/Divinylbenzol-Copolymeren basiert.

19. Verfahren gemäß einem der Ansprüche 11 oder 14 bis 18, **dadurch gekennzeichnet, daß** das/die Übergangsmetall(e) ausgewählt ist/sind aus der Gruppe bestehend aus Cu, Ag, Au, Zn, Cd, Hg, V, Nb, Ta, Cr, Mo, W, Fe, Ru, Os, Co, Rh, Ir, Ni, Pd, Pt, vorzugsweise aus der Gruppe bestehend aus Ru, Rh, Pd, Os, Ir und Pt, besonders bevorzugt aus der Gruppe bestehend aus Pd, Ru, Pt und Ir, ganz besonders bevorzugt Pd ist.

20. Verfahren gemäß einem der Ansprüche 11 oder 14 bis 19, **dadurch gekennzeichnet, daß** ein oder mehrere Übergangsmetalle in der gleichen oder verschiedenen Oxidationsstufen vorliegen, gegebenenfalls vorzugsweise in jeweils wenigstens zwei verschiedenen Oxidationsstufen vorliegen.

21. Verfahren zur Herstellung wenigstens einer substituierten Dimethyl-(3-aryl-butyl)-amin-verbindung der allgemeinen Formel III, 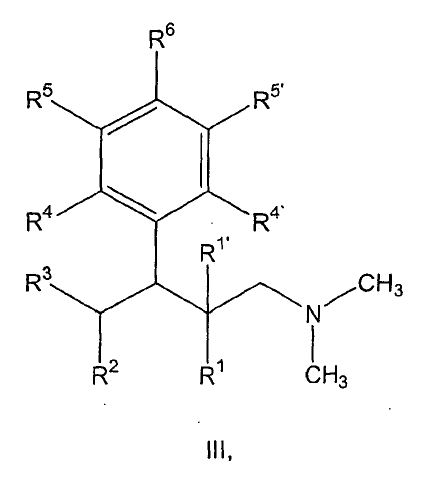 worin die Reste R¹, R¹', R², R³, R⁴, R^{4'}, R⁵, R^{5'} und R⁶ die Bedeutung gemäß Anspruch 1 haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salze, oder jeweils in Form eines Solvates, **dadurch gekennzeichnet, daß** wenigstens eine substituierte Dimethyl-(3-aryl-but-3-enyl)-amin-Verbindung der allgemeinen Formel II 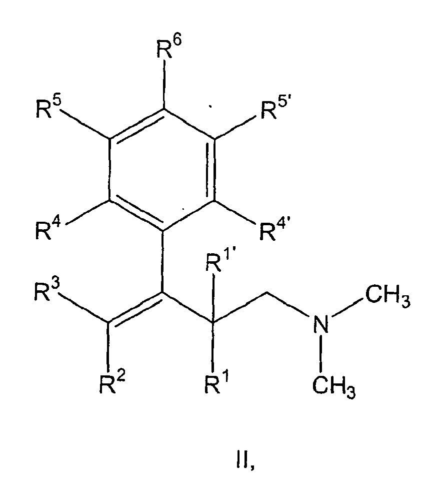 worin die Reste R¹, R¹', R², R³, R⁴, R^{4'}, R⁵, R^{5'} und R⁶ die Bedeutung gemäß Anspruch 1 haben, jeweils in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form eines physiologisch verträglichen Salzes, oder jeweils in Form eines Solvates, durch Hydrierung mit Wasserstoff unter heterogener Katalyse in Gegenwart eines Gemisches aus wenigstens einem Katalysator gemäß den Ansprüchen 5 bis 10 und wenigstens einem Katalysator, der ein oder mehrere Übergangsmetalle aufweist, oder in Gegenwart wenigstens eines bifunktionalisierten Katalysators gemäß den Ansprüchen 14 bis 20 zu einer Verbindung der allgemeinen Formel III als Endprodukt umgesetzt wird.

22. Verfahren gemäß Anspruch 21, **dadurch gekennzeichnet, daß** das/die Übergangsmetall(e) ausgewählt ist/sind aus der Gruppe bestehend aus Cu, Ag, Au, Zn, Cd, Hg, V, Nb, Ta, Cr, Mo, W, Fe, Ru, Os, Co, Rh, Ir, Ni, Pd, Pt, vorzugsweise aus der Gruppe bestehend aus Ru, Rh, Pd, Os, Ir und Pt, besonders bevorzugt aus der Gruppe bestehend aus Pd, Ru, Pt und Ir, ganz besonders bevorzugt Pd ist.

23. Verfahren gemäß Anspruch 21 oder 22, **dadurch gekennzeichnet, daß** ein oder mehrere Übergangsmetalle in der gleichen oder verschiedenen Oxidationsstufen vorliegen, gegebenenfalls vorzugsweise in jeweils wenigstens zwei verschiedenen Oxidationsstufen vorliegen.

24. Verfahren gemäß einem der Ansprüche 1 bis 23, **dadurch gekennzeichnet, daß** die jeweilige substituierte 4-Dimethylamino-2-aryl-butan-2-ol-Komponente und/oder die jeweilige substituierte Dimethyl-(3-aryl-but-3-enyl)-amin-Komponente mit einem Reaktionsmedium gemischt oder darin gelöst vorliegen, welches unter den jeweiligen Reaktionsbedingungen flüssig ist.

25. Verfahren gemäß Anspruch 24, **dadurch gekennzeichnet, daß** das Reaktionsmedium auf Wasser, auf einer oder mehrerer organischer Verbindungen oder auf einem Gemisch aus Wasser und einer oder mehrerer organischer Verbindungen basiert.

26. Verfahren gemäß Anspruch 25, **dadurch gekennzeichnet, daß** die organische Verbindung ausgewählt ist aus der Gruppe bestehend aus halogenierten organischen Verbindungen, Alkoholen und Ketonen, vorzugsweise aus der Gruppe bestehend aus Dichlormethan, Chloroform, Toluol, Methanol, Ethanol, Aceton, besonders bevorzugt Methanol und/oder Ethanol ist.

27. Verfahren gemäß einem der Ansprüche 1 bis 26, **dadurch gekennzeichnet, daß** die Dehydratisierung zum Zwischenprodukt und/oder die Umsetzung zum Endprodukt jeweils bei Temperaturen von 20-250°C, vorzugsweise 50-180°C, besonders bevorzugt 100-160° C durchgeführt wird.

28. Verfahren gemäß einem der Ansprüche 1 bis 27, **dadurch gekennzeichnet, daß** die Dehydratisierung zum Zwischenprodukt und/oder die Umsetzung zum Endprodukt bei einem Druck von 0,01 bis 300 bar, vorzugsweise 2 bis 10 bar durchgeführt werden.

29. Verfahren gemäß einem der Ansprüche 1 bis 28, **dadurch gekennzeichnet, daß** die Dehydratisierung zum Zwischenprodukt und/oder die Umsetzung zum Endprodukt diskontinuierlich, vorzugsweise in einem slurry-Reaktor, durchgeführt wird.

30. Verfahren gemäß einem der Ansprüche 1 bis 28, **dadurch gekennzeichnet, daß** die Dehydratisierung zum Zwischenprodukt und/oder die Umsetzung zum Endprodukt kontinuierlich, vorzugsweise in einem Festbettreaktor oder in einem Schlaufenreaktor, durchgeführt wird.

## Claims

1. Process for the dehydration of at least one substituted 4-dimethylamino-2-aryl-butan-2-ol compound of the general formula I 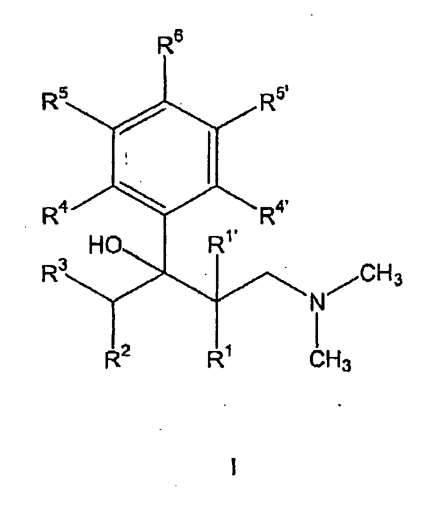 wherein
R' represents H or C₁₋₅-alkyl,
R^{1'} represents H or C₁₋₅-alkyl,
R² represents H or C₁₋₅-alkyl,
R³ represents H or C₁₋₅-alkyl,
R⁴, R^{4'}, R⁵, R^{5'}, R⁶, which may be identical or different, each represents H, OH, C₁₋₄-alkyl, OC₁₋₄-alkyl, partially fluorinated or perfluorinated C₁₋₄-alkyl, partially fluorinated or perfluorinated O-C₁₋₄-alkyl, O-(CH₂)ₙ-phenyl where n = 1, 2 or 3, F, Cl or OR⁸,
or two adjacent radicals R⁴ and R⁵, R⁵ and R⁶, R⁶ and R^{5'} or R^{5'} and R^{4'} represent a group -OCH=CHO-, -CH=C(R⁹)-O-, -CH=C(R⁹)-S- or -CH=CH-C(OR¹⁰)=CH- as part of a ring, with the proviso that the other radicals in each case R⁶, R^{5'} and R^{4'}, R⁴, R^{5'} and R^{6'}, R⁴, R⁵ and R^{4'} or R⁴, R⁵ and R⁶ are as defined above,
R⁸ represents CO-C₁₋₅-alkyl, PO(O-C₁₋₄-alkyl)₂, CO-C₆H₄-R¹¹, CO(O-C₁₋₅-alkyl), CO-CHR¹²-NHR¹³, CO-NH-C₆H₃-(R¹⁴)₂ or an unsubstituted or at least monosubstituted pyridyl, thienyl, thiazoyl or phenyl group,
R⁹ represents H or C₁₋₄-alkyl,
R¹⁰ represents H or C₁₋₃-alkyl,
R¹¹ represents OC(O)-C₁₋₃-alkyl in the ortho-position or -CH₂-N-(R₁₅)₂ in the meta- or para-position, where R¹⁵ in each case represents C₁₋₄-alkyl or the two radicals R¹⁵, together with the bridging nitrogen atom, form a 4-morpholino radical,
R¹² and R¹³, which may be identical or different, each represents H, C₁₋₆-alkyl or C₃₋₈-cycloalkyl,
or R¹² and R¹³ together represent -(CH₂)₃₋₈,
R¹⁴ represents H, OH, C₁₋₇-alkyl, partially fluorinated or perfluorinated C₁₋₇-alkyl, OC₁₋₇-alkyl, phenyl, O-aryl, F or Cl, with the proviso that the radicals R¹⁴ are identical or different,
in each case in the form of one of their pure stereoisomers, in particular enantiomers or diastereoisomers, of their racemates or in the form of a mixture of stereoisomers, in particular of enantiomers or diastereoisomers, in any desired mixing ratio, or in each case in the form of a physiologically acceptable salt, or in each case in the form of a solvate,
**characterised in that** it is dehydrated with heterogeneous catalysis to form a substituted dimethyl-(3-aryl-but-3-enyl)-amine compound of the general formula II 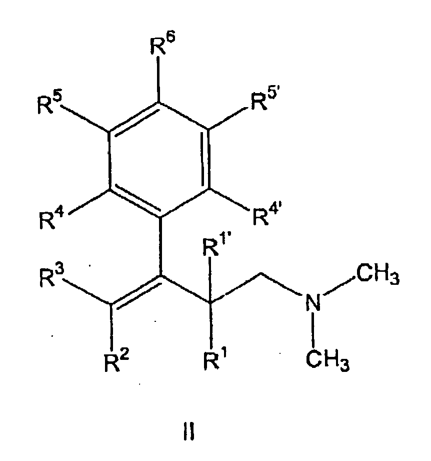 wherein R¹, R^{1'}, R², R³, R⁴, R^{4'}, R⁵, R^{5'} and R⁶ are as defined above, in each case optionally in the form of one of their pure stereoisomers, in particular enantiomers or diastereoisomers, of their racemates or in the form of a mixture of stereoisomers, in particular of enantiomers or diastereoisomers, in any desired mixing ratio, or in each case in the form of a physiologically acceptable salt, or in each case in the form of a solvate.

2. Process for the preparation of at least one substituted dimethyl-(3-aryl-butyl)-amine compound of the general formula III 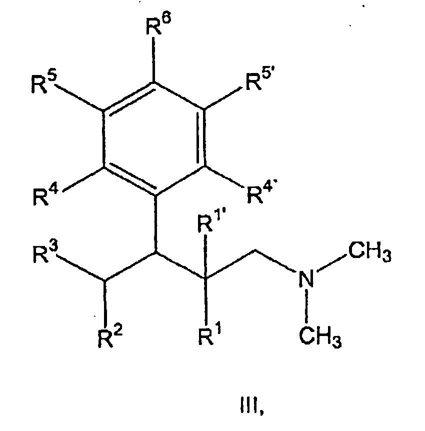 wherein the radicals R¹, R^{1'}, R², R³, R⁴, R^{4'}, R⁵, R^{5'} and R⁶ are as defined in claim 1, in each case in the form of one of their pure stereoisomers, in particular enantiomers or diastereoisomers, of their racemates or in the form of a mixture of stereoisomers, in particular of enantiomers or diastereoisomers, in any desired mixing ratio, or in each case in the form of a physiologically acceptable salt, or in each case in the form of a solvate, **characterised in that** at least one substituted 4-dimethylamino-2-aryl-butan-2-ol compound of the general formula I 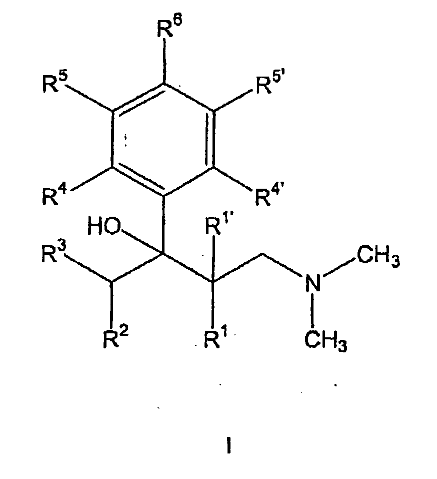 wherein the radicals R¹, R^{1'}, R², R³, R⁴, R^{4'}, R⁵, R^{5'} and R⁶ are as defined in claim 1, in each case in the form of one of their pure stereoisomers, in particular enantiomers or diastereoisomers, of their racemates or in the form of a mixture of stereoisomers, in particular of enantiomers or diastereoisomers, in any desired mixing ratio, or in each case in the form of a physiologically acceptable salt, or in each case in the form of a solvate, is dehydrated with heterogeneous catalysis to form, as intermediate, a substituted dimethyl-(3-aryl-but-3-enyl)-amine compound of the general formula II 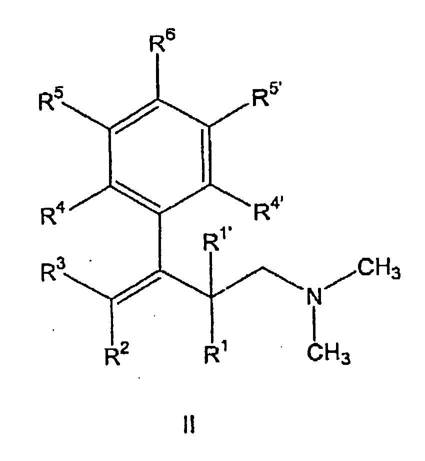 wherein the radicals R¹, R^{1'}, R², R³, R⁴, R^{4'}, R⁵, R^{5'} and R⁶ are as defined in claim 1, in each case in the form of one of their pure stereoisomers, in particular enantiomers or diastereoisomers, of their racemates or in the form of a mixture of stereoisomers, in particular of enantiomers or diastereoisomers, in any desired mixing ratio, or in each case in the form of a physiologically acceptable salt, or in each case in the form of a solvate, the intermediate is optionally isolated, is optionally purified and is reacted to form a dimethyl-(3-aryl-butyl)-amine compound of the general formula III as end product.

3. Process according to claim 2, **characterised in that** the reaction to form the compound of the general formula III is carried out with heterogeneous catalysis.

4. Process according to claim 2 or 3, **characterised in that** the reaction to form the compound of the general formula III is carried out by hydrogenation with hydrogen.

5. Process according to any one of claims 1 to 4, **characterised in that** the dehydration is carried out in the presence of at least one acidic catalyst and/or at least one basic catalyst, preferably in the presence of at least one acidic catalyst.

6. Process according to claim 5, **characterised in that** the acidic catalyst(s) is/are selected from the group consisting of ion-exchange resins, zeolites, heteropoly acids, phosphates, sulfates and optionally mixed metal oxides.

7. Process according to claim 6, **characterised in that** the ion-exchange resin contains sulfonic acid groups.

8. Process according to claim 6 or 7, **characterised in that** the ion-exchange resin is based on at least one tetrafluoroethylene/perfluorovinyl ether copolymer.

9. Process according to claim 6 or 7, **characterised in that** the ion-exchange resin is based on at least one styrene/divinylbenzene copolymer.

10. Process according to claim 6, **characterised in that** a metal oxide selected from the group consisting of SiO₂, Al₂O₃, TiO₂, Nb₂O₅ and B₂O₃ and/or Al₂O₃/SiO₂ and/or Al₂O₃/B₂O₃ is present as mixed metal oxide.

11. Process according to any one of claims 3 to 10, **characterised in that** the reaction to form the compound of the general formula III is carried out in the presence of at least one catalyst that contains one or more transition metals and optionally in the presence of at least one catalyst according to claims 5 to 10.

12. Process according to any one of claims 3 to 10, **characterised in that** the reaction to form the end product is carried out in the presence of at least one polyfunctionalised, preferably bifunctionalised, catalyst.

13. Process according to claim 3 or 4, **characterised in that** the dehydration to form the intermediate and the reaction thereof without purification and/or isolation to form the end product are carried out in the presence of at least one polyfunctionalised, preferably bifunctionalised, catalyst.

14. Process according to claim 12 or 13, **characterised in that** there is used as bifunctionalised catalyst a catalyst that is acidic and/or basic, preferably acidic, and contains one or more transition metals.

15. Process according to claim 14, **characterised in that** there is used as acidic catalyst at least one ion-exchange resin that contains one or more transition metals.

16. Process according to claim 15, **characterised in that** the ion-exchange resin contains sulfonic acid groups.

17. Process according to claim 15 or 16, **characterised in that** the ion-exchange resin is based on at least one tetrafluoroethylene/perfluorovinyl ether copolymer.

18. Process according to claim 15 or 16, **characterised in that** the ion-exchange resin is based on at least one styrene/divinylbenzene copolymer.

19. Process according to any one of claims 11 or 14 to 18, **characterised in that** the transition metal(s) is/are selected from the group consisting of Cu, Ag, Au, Zn, Cd, Hg, V, Nb, Ta, Cr, Mo, W, Fe, Ru, Os, Co, Rh, Ir, Ni, Pd, Pt, preferably from the group consisting of Ru, Rh, Pd, Os, Ir and Pt, particularly preferably from the group consisting of Pd, Ru, Pt and Ir, Pd being very particularly preferred.

20. Process according to any one of claims 11 or 14 to 19, **characterised in that** one or more transition metals are present in the same or different oxidation states, optionally preferably in at least two different oxidation states.

21. Process for the preparation of at least one substituted dimethyl-(3-aryl-butyl)-amine compound of the general formula III 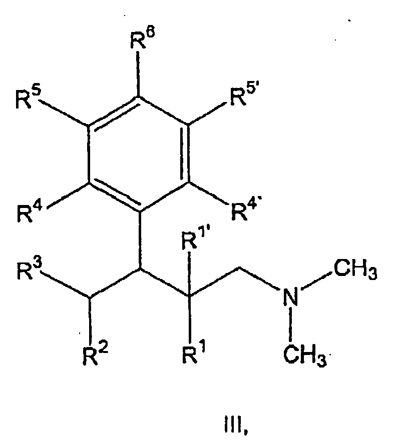 wherein the radicals R¹, R^{1'}, R², R³, R⁴, R^{4'}, R⁵, R^{5'} and R⁶ are as defined in claim 1, in each case in the form of one of their pure stereoisomers, in particular enantiomers or diastereoisomers, of their racemates or in the form of a mixture of stereoisomers, in particular of enantiomers or diastereoisomers, in any desired mixing ratio, or in each case in the form of a physiologically acceptable salt, or in each case in the form of a solvate, **characterised in that** at least one substituted dimethyl-(3-aryl-but-3-enyl)-amine compound of the general formula II 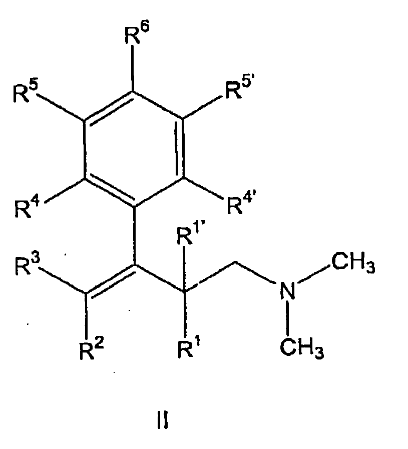 wherein the radicals R¹, R^{1'}, R², R³, R⁴, R^{4'}, R⁵, R^{5'} and R⁶ are as defined in claim 1, in each case in the form of one of their pure stereoisomers, in particular enantiomers or diastereoisomers, of their racemates or in the form of a mixture of stereoisomers, in particular of enantiomers or diastereoisomers, in any desired mixing ratio, or in each case in the form of a physiologically acceptable salt, or in each case in the form of a solvate, is reacted to form a compound of the general formula III as end product by hydrogenation with hydrogen with heterogeneous catalysis in the presence of a mixture comprising at least one catalyst according to claims 5 to 10 and at least one catalyst containing one or more transition metals, or in the presence of at least one bifunctionalised catalyst according to claims 14 to 20.

22. Process according to claim 21, **characterised in that** the transition metal(s) is/are selected from the group consisting of Cu, Ag, Au, Zn, Cd, Hg, V, Nb, Ta, Cr, Mo, W, Fe, Ru, Os, Co, Rh, Ir, Ni, Pd, Pt, preferably from the group consisting of Ru, Rh, Pd, Os, Ir and Pt, particularly preferably from the group consisting of Pd, Ru, Pt and Ir, Pd being very particularly preferred.

23. Process according to claim 21 or 22, **characterised in that** one or more transition metals are present in the same or different oxidation states, optionally preferably in at least two different oxidation states.

24. Process according to any one of claims 1 to 23, **characterised in that** the substituted 4-dimethylamino-2-aryl-butan-2-ol component and/or the substituted dimethyl-(3-aryl-but-3-enyl)-amine component is mixed with or dissolved in a reaction medium that is liquid under the particular reaction conditions.

25. Process according to claim 24, **characterised in that** the reaction medium is based on water, on one or more organic compounds or on a mixture of water and one or more organic compounds.

26. Process according to claim 25, **characterised in that** the organic compound is selected from the group consisting of halogenated organic compounds, alcohols and ketones, preferably from the group consisting of dichloromethane, chloroform, toluene, methanol, ethanol, acetone, particularly preferably is methanol and/or ethanol.

27. Process according to any one of claims 1 to 26, **characterised in that** the dehydration to form the intermediate and/or the reaction to form the end product is/are each carried out at temperatures of from 20 to 250°C, preferably from 50 to 180°C, particularly preferably from 100 to 160°C.

28. Process according to any one of claims 1 to 27, **characterised in that** the dehydration to form the intermediate and/or the reaction to form the end product is/are carried out at a pressure of from 0.01 to 300 bar, preferably from 2 to 10 bar.

29. Process according to any one of claims 1 to 28, **characterised in that** the dehydration to form the intermediate and/or the reaction to form the end product is/are carried out discontinuously, preferably in a slurry reactor.

30. Process according to any one of claims 1 to 28, **characterised in that** the dehydration to form the intermediate and/or the reaction to form the end product is/are carried out continuously, preferably in a fixed-bed reactor or in a loop reactor.

## Revendications

1. Procédé de déshydratation d'au moins un composé substitué de 4-diméthylamino-2-aryl-butane-2-ol de formule générale I 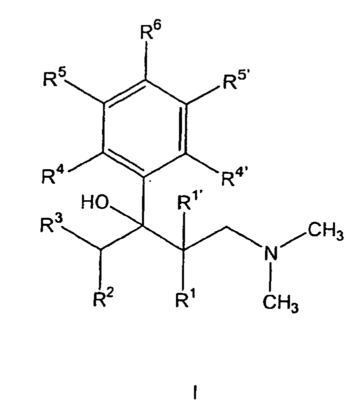 dans laquelle
R¹ représente H ou alkyle en C₁₋₅,
R^{1'} représente H ou alkyle en C₁₋₅,
R² représente H ou alkyle en C₁₋₅,
R³ représente H ou alkyle en C₁₋₅,
R⁴, R^{4'}, R⁵, R^{5'}, R⁶, identiques ou différents, représentent chacun H, OH, alkyle en C₁₋₄, O-alkyle en C₁₋₄, alkyle en C₁₋₄ partiellement ou entièrement fluoré, O-alkyle en C₁₋₄ partiellement ou entièrement fluoré, O-(CH₂)ₙ-phényle avec n = 1, 2 ou 3, F, Cl ou OR⁸, ou bien deux restes contigus R⁴ et R⁵, R⁵ et R⁶, R⁶ et R^{5'} ou R^{5'} et R^{4'} représentent un groupe -OCH=CHO-, -CH=C(R⁹)-O-, -CH=C(R⁹)-S- ou -CH=CH-C(OR¹⁰)=CH- forment une partie d'un noyau, sous réserve qu'à chaque fois les autres restes R⁶, R^{5'} et R^{4'}, R⁴, R^{5'} et R^{6'}, R⁴, R⁵ et R^{4'} et respectivement R⁴, R⁵ et R⁶ aient la définition mentionnée ci-dessus,
R⁸ représente CO-(alkyle en C₁₋₅), PO(O-alkyle en C₁₋₄)₂, CO-C₆H₄-R¹¹, CO(O-alkyle en C₁₋₅), CO-CHR¹²-NHR¹³, CO-NH-C₆H₃-(R¹⁴)₂ ou un groupe pyridyle, thiényle, thiazolyle ou phényle non substitué ou tout au moins simplement substitué, R⁹ représente H ou alkyle en C₁₋₄,
R¹⁰ représente H ou alkyle en C₁₋₃,
R¹¹ représente OC(O)-(alkyle en C₁₋₃) en position ortho ou -CH₂-N-(R₁₅)₂ en position méta ou para, chaque R¹⁵ étant un alkyle en C₁₋₄ ou les deux restes R¹⁵ formant un reste 4-morpholino conjointement avec l'atome d'azote de pontage,
R¹² et R¹³ identiques ou différents représentent chacun H, alkyle en C₁₋₆ ou cycloalkyle en C₃₋₈,
ou bien R¹² et R¹³ représentent conjointement -(CH₂)₃₋₈,
R¹⁴ représente H, OH, alkyle en C₁₋₇, alkyle en C₁₋₇ partiellement ou entièrement fluoré, O-alkyle en C₁₋₇, phényle, O-aryle, F, Cl, sous réserve que les restes R¹⁴ soient identiques ou différents,
dans chaque cas sous forme d'un de ses stéréoisomères purs, en particulier d'énantiomères ou de diastéréoisomères, de ses racémates ou sous forme d'un mélange de stéréoisomères, en particulier des énantiomères ou des diastéréoisomères, dans un rapport de mélange quelconque, ou dans chaque cas sous forme d'un sel physiologiquement acceptable, ou dans chaque cas sous forme d'un produit de solvatation,
**caractérisé en ce qu'**il est déshydraté sous catalyse hétérogène en un composé substitué de diméthyl-(3-aryl-but-3-ényl)-amine de formule générale II 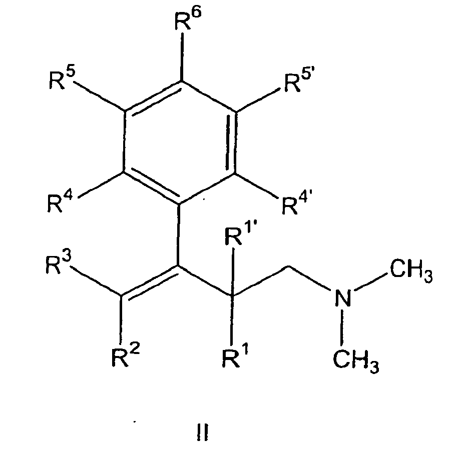 dans laquelle R¹, R^{1'}, R², R³, R⁴, R^{4'}, R⁵, R^{5'} et R⁶ ont la définition indiquée ci-dessus, à chaque fois le cas échéant sous forme d'un de ses stéréoisomère purs, en particulier d'énantiomères ou de diastéréoisomères, de ses racémates ou sous forme d'un mélange de stéréoisomères, en particulier des énantiomères ou des diastéréoisomères, dans un rapport de mélange quelconque, ou dans chaque cas sous forme d'un sel physiologiquement acceptable, ou dans chaque cas sous forme d'un produit de solvatation.

2. Procédé de production d'au moins un composé substitué de diméthyl-(3-aryl-butyl)-amine de formule générale III 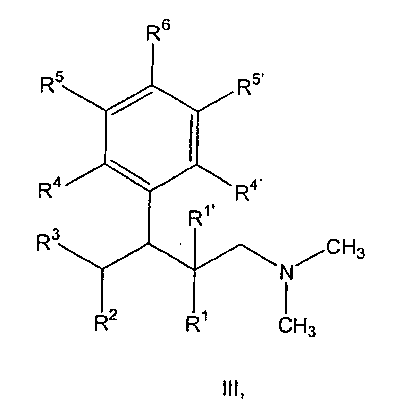 dans laquelle les restes R¹, R^{1'}, R², R³, R⁴, R^{4'}, R⁵, R^{5'} et R⁶ ont la définition selon la revendication 1, dans chaque cas sous forme d'un de ses stéréoisomères purs, en particulier d'énantiomères ou de diastéréoisomères, de ses racémates ou sous forme d'un mélange de stéréoisomères, en particulier des énantiomères ou des diastéréoisomères, dans un rapport de mélange quelconque, ou dans chaque cas sous forme d'un sel physiologiquement acceptable, ou dans chaque cas sous forme d'un produit de solvatation, **caractérisé en ce qu'**au moins un composé substitué de 4-diméthylamino-2-aryl-butane-2-ol de formule générale I 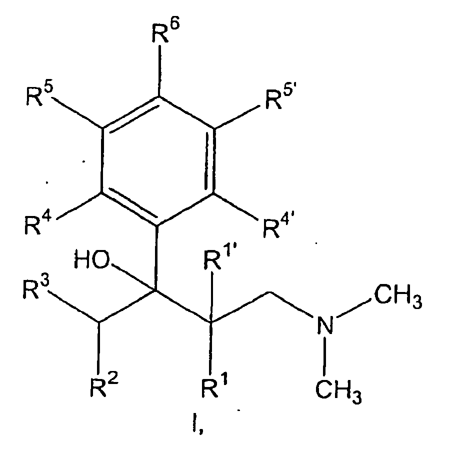 dans laquelle les restes R¹, R^{1'}, R², R³, R⁴, R^{4'}, R⁵, R^{5'} et R⁶ ont la définition selon la revendication 1, dans chaque cas sous forme d'un de ses stéréoisomère purs, en particulier d'énantiomères ou de diastéréoisomères, de ses racémates ou sous forme d'un mélange de stéréoisomères, en particulier des énantiomères ou des diastéréoisomères, dans un rapport de mélange quelconque, ou dans chaque cas sous forme d'un sel physiologiquement acceptable, ou dans chaque cas sous forme d'un produit der solvatation, est déshydraté comme produit intermédiaire sous catalyse hétérogène en un composé substitué de diméthyl-(3-aryl-but-3-ényl)-amine de formule générale II 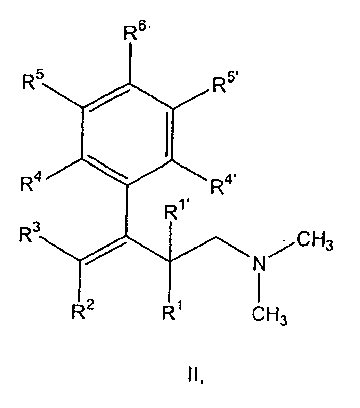 dans laquelle les restes R¹, R^{1'}, R², R³, R⁴, R^{4'}, R⁵, R^{5'} et R⁶ ont la définition indiquée dans la revendication 1, à chaque fois sous forme d'un de ses stéréoisomère purs, en particulier d'énantiomères ou de diastéréoisomères, de ses racémates ou sous forme d'un mélange de stéréoisomères, en particulier des énantiomères ou des diastéréoisomères, dans un rapport de mélange quelconque, ou dans chaque cas sous forme d'un sel physiologiquement acceptable, ou dans chaque cas sous forme d'un produit de solvatation, ce produit intermédiaire est éventuellement isolé, éventuellement purifié et transformé en un composé de diméthyl-(3-aryl-butyl)-amine de formule générale III comme produit final.

3. Procédé suivant la revendication 2, **caractérisé en ce que** la transformation en composé de formule générale III s'effectue sous catalyse hétérogène.

4. Procédé suivant la revendication 2 ou 3, **caractérisé en ce que** la transformation en composé de formule générale III s'effectue par hydrogénation avec l'hydrogène.

5. Procédé suivant l'une des revendications 1 à 4, **caractérisé en ce que** la déshydratation s'effectue en présence d'au moins un catalyseur acide et/ou d'au moins un catalyseur basique, avantageusement en présence d'au moins un catalyseur acide.

6. Procédé suivant la revendication 5, **caractérisé en ce que** le/les catalyseur(s) acide(s) est(sont) choisi(s) dans le groupe constitué de résines échangeuses d'ions, de zéolites, d'hétéropolyacides, de phosphates, de sulfates et le cas échéant d'oxydes métalliques en mélange.

7. Procédé suivant la revendication 6, **caractérisé en ce que** la résine échangeuse d'ions porte des groupes acide sulfonique.

8. Procédé suivant la revendication 6 ou 7, **caractérisé en ce que** la résine échangeuse d'ions est à base d'au moins un copolymère tétrafluoréthylène/éther de perfluorovinyle.

9. Procédé suivant la revendication 6 ou 7, **caractérisé en ce que** la résine échangeuse d'ions est à base d'un moins un copolymère styrène/divinylbenzène.

10. Procédé suivant la revendication 6, **caractérisé en ce qu'**un oxyde métallique choisi dans le groupe constitué de SiO₂, Al₂O₃, TiO₂, Nb₂O₅ et B₂O₃ et/ou Al₂O₃/SiO₂ et/ou Al₂O₃/B₂O₃ est présent comme oxyde métallique en mélange.

11. Procédé suivant l'une des revendications 3 à 10, **caractérisé en ce que** la transformation en composé de formule générale III s'effectue en présence d'au moins un catalyseur qui comprend un ou plusieurs métaux de transition et éventuellement en présence d'au moins un catalyseur suivant les revendications 5 à 10.

12. Procédé suivant l'une des revendications 3 à 10, **caractérisé en ce que** la transformation en produit final s'effectue en présence d'au moins un catalyseur polyfonctionnalisé, avantageusement bifonctionnalisé.

13. Procédé suivant la revendication 3 ou 4, **caractérisé en ce que** la déshydratation en produit intermédiaire et sa transformation s'effectuent sans purification et/ou isolement en produit final en présence d'au moins un catalyseur polyfonctionnalisé, avantageusement bifonctionnalisé.

14. Procédé suivant la revendication 12 ou 13, **caractérisé en ce qu'**un catalyseur qui est acide et/ou basique, avantageusement acide, et contient un ou plusieurs métaux de transition, est utilisé comme catalyseur bifonctionnalisé.

15. Procédé suivant la revendication 14, **caractérisé en ce qu'**au moins une résine échangeuse d'ions qui contient un ou plusieurs métaux de transition est utilisée comme catalyseur acide.

16. Procédé suivant la revendication 15, **caractérisé en ce que** la résine échangeuse d'ions porte des groupes acide sulfonique.

17. Procédé suivant la revendication 15 ou 16, **caractérisé en ce que** la résine échangeuse d'ions est à base d'au moins un copolymère tétrafluoréthylène/éther de perfluorovinyle.

18. Procédé suivant la revendication 15 ou 16, **caractérisé en ce que** la résine échangeuse d'ions est à base d'au moins un copolymère styrène/divinylbenzène.

19. Procédé suivant l'une des revendications 11 ou 14 à 18, **caractérisé en ce que** le/les métal/métaux de transition est/sont choisi(s) dans le groupe constitué de Cu, Ag, Au, Zn, Cd, Hg, V, Nb, Ta, Cr, Mo, W, Fe, Ru, Os, Co, Rh, Ir, Ni, Pd, Pt, avantageusement dans le groupe constitué de Ru, Rh, Pd, Os, Ir et Pt, très avantageusement dans le groupe constitué de Pd, Ru, Pt et Ir, Pd étant tout particulièrement apprécié.

20. Procédé suivant l'une des revendications 11 ou 14 à 19, **caractérisé en ce qu'**un ou plusieurs métaux de transition sont présents aux mêmes degrés d'oxydation ou à des degrés différents, le cas échéant avantageusement présents dans chaque cas à au moins deux degrés d'oxydation différents.

21. Procédé de production d'au moins un composé substitué de diméthyl-(3-aryl-butyl)-amine de formule générale III 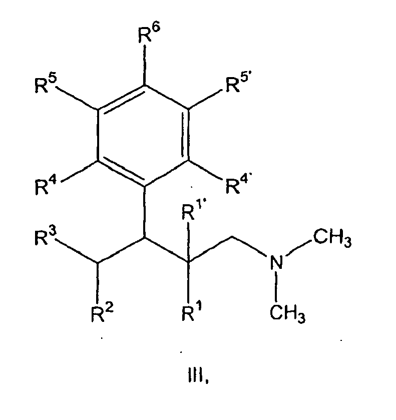 dans laquelle les restes R¹, R^{1'}, R², R³, R⁴, R^{4'}, R⁵, R^{5'} et R⁶ ont la définition selon la revendication 1, dans chaque cas sous forme d'un de ses stéréoisomères purs, en particulier d'énantiomères ou de diastéréoisomères, de ses racémates ou sous forme d'un mélange de stéréoisomères, en particulier des énantiomères ou des diastéréoisomères, dans un rapport de mélange quelconque, ou dans chaque cas sous forme d'un sel physiologiquement acceptable, ou dans chaque cas sous forme d'un produit der solvatation, **caractérisé en ce qu'**au moins un composé substitué de diméthyl-(3-aryl-but-3-ényl)-amine de formule générale II 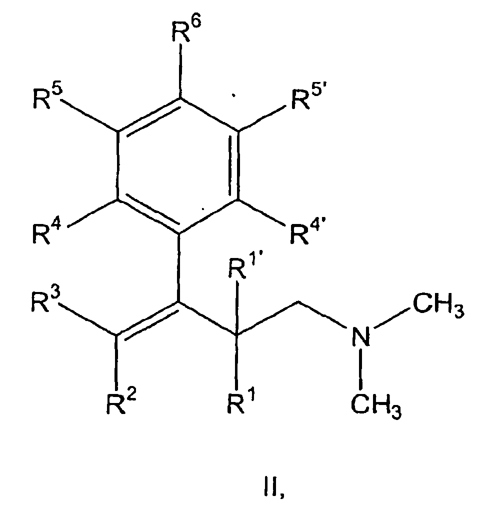 dans laquelle les restes R¹, R^{1'}, R², R³, R⁴, R^{4'}, R⁵, R^{5'} et R⁶ ont la définition selon la revendication 1, dans chaque cas sous forme d'un de ses stéréoisomères purs, en particulier d'énantiomères ou de diastéréoisomères, de ses racémates ou sous forme d'un mélange de stéréoisomères, en particulier des énantiomères ou des diastéréoisomères, dans un rapport de mélange quelconque, ou dans chaque cas sous forme d'un sel physiologiquement acceptable, ou dans chaque cas sous forme d'un produit de solvatation, est transformé par hydrogénation avec l'hydrogène sous catalyse hétérogène en présence d'un mélange d'au moins un catalyseur selon les revendications 5 à 10 et d'au moins un catalyseur qui contient un ou plusieurs métaux de transition, ou en présence d'au moins un catalyseur bifonctionnalisé selon les revendications 14 à 20, en un composé de formule générale III comme produit final.

22. Procédé suivant la revendication 21, **caractérisé en ce que** le/les métal/métaux de transition est/sont choisi(s) dans le groupe constitué de Cu, Ag, Au, Zn, Cd, Hg, V, Nb, Ta, Cr, Mo, W, Fe, Ru, Os, Co, Rh, Ir, Ni, Pd, Pt, avantageusement dans le groupe constitué de Ru, Rh, Pd, Os, Ir et Pt, très avantageusement dans le groupe constitué de Pd, Ru, Pt et Ir, Pd étant tout particulièrement apprécié.

23. Procédé suivant la revendication 21 ou 22, **caractérisé en ce qu'**un ou plusieurs métaux de transition sont présents aux mêmes degrés d'oxydation ou à des degrés différents, le cas échéant avantageusement présents dans chaque cas à au moins deux degrés d'oxydation différents.

24. Procédé suivant l'une des revendications 1 à 23, **caractérisé en ce que** le composant 4-diméthylamino-2-aryl-butane-2-ol substitué respectif et/ou le composant diméthyl-(3-aryl-but-3-ényl)-amine substituée respectif sont présents en mélange avec ou en solution dans un milieu de réaction qui est liquide dans les conditions réactionnelles correspondantes.

25. Procédé suivant la revendication 24, **caractérisé en ce** le milieu de réaction est à base d'eau, d'un ou plusieurs composés organiques ou d'un mélange d'eau et d'un ou plusieurs composés organiques.

26. Procédé suivant la revendication 25, **caractérisé en ce que** le composé organique est choisi dans le groupe constitué de composés organiques halogénés, d'alcools et de cétones, avantageusement dans le groupe constitué de diclorométhane, de chloroforme, de toluène, de méthanol, d'éthanol, d'acétone, très avantageusement de méthanol et/ou d'éthanol.

27. Procédé suivant l'une des revendications 1 à 26, **caractérisé en ce que** la déshydratation en produit intermédiaire et/ou la transformation en produit final sont conduites chacune à des températures de 20-250°C, avantageusement 50-180°C, très avantageusement 100-160°C.

28. Procédé suivant l'une des revendications 1 à 27, **caractérisé en ce que** la déshydratation en produit intermédiaire et/ou la transformation en produit final sont conduites à une pression de 0,01 à 300 bar, avantageusement de 2 à 10 bar.

29. Procédé suivant l'une des revendications 1 à 28, **caractérisé en ce que** la déshydratation en produit intermédiaire et/ou la transformation en produit final sont conduites en discontinu, avantageusement dans un réacteur à charge en suspension.

30. Procédé suivant l'une des revendications 1 à 28, **caractérisé en ce que** la déshydratation en produit intermédiaire et/ou la transformation en produit final sont conduites en continu, avantageusement dans un réacteur à lit fixe ou dans un réacteur à écoulement en boucle.
